# EUROPEAN PATENT APPLICATION

(11) **EP 2 112 145 A1**
(43) Date of publication of application: **28.10.2009**
(21) Application number: 08155123.6
(22) Date of filing: 24.04.2008
(51) Int. Cl.: C07D 311/28, C07D 493/04, A61K 31/352, A61P 25/28

(54) **Chromenone derivatives useful for the treatment of neurodegenerative diseases**

(71) Applicant: AxoGlia Therapeutics S.A., 1316 Luxembourg (LU)
(72) Inventor: Coowar, Djalil, F-57000 Metz (FR); Couche, Emmanuel, L-4930 Bascharage (LU); Koncina, Eric, F-57100 Thionville (FR)
(74) Representative: Office Freylinger

(57) **Abstract**

Compounds of general formula (I) and (II) 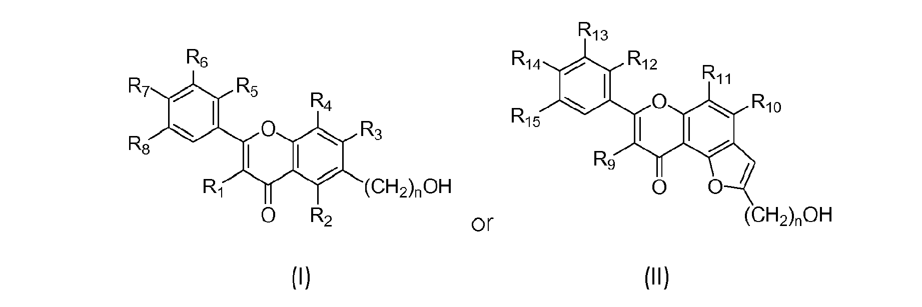 in which R₁, R₂, R₃, R₄, R₅, R₆, R₇, R₈, R₉, R₁₀, R₁₁, R₁₂, R₁₃, R₁₄ and R₁₅ have the meanings given in the specification, are useful in the treatment of neurodegenerative disease.

## Description

### FIELD OF THE INVENTION

The present invention relates to chromenone derivatives, to a process for preparing the chromenone derivatives, to a pharmaceutical composition comprising the chromenone derivatives and to the use of the chromenone derivatives in the treatment of neurodegenerative disease.

### BACKGROUND OF THE INVENTION

Neural cell death is one of the major characteristics of degenerative neuropathologies like Alzheimer's disease (AD), Parkinson disease (PD), Multiple sclerosis (MS) or Stroke, although loss of cell functions of neurons and/or glial cells is also known to contribute to these degenerative diseases. Adult mammalian brain has limited capacity for regeneration. This makes the repair of any injuries hazardous and, consequently, CNS traumas are devastating.

Alzheimer's disease (AD) is the most common cause of dementia that gradually destroys neurons and affects more than 24 million people worldwide. It occurs mostly in older adults and patients afflicted with AD lose their ability to learn, remember, make decisions, communicate and carry out daily activities. The cause and progression of AD is not well understood, but is associated with amyloid beta (Aβ) plaques and neurofibrillary tangles in the brain.

Parkinson's disease (PD) is a degenerative disorder of the central nervous system affecting more than 6 million people worldwide and that often impairs the sufferer's motor skills and speech. The symptoms of Parkinson's disease result from the loss of dopamine-secreting cells in the region of the substantia nigra (literally "black substance"). These neurons project to the striatum and their loss leads to alterations in the activity of the neural circuits within the basal ganglia that regulate movement.

Multiple sclerosis (MS) is a neurological disease of the young adult which associates demyelination with inflammatory or even immunological elements. For the majority of patients, MS is known to evolve initially by "relapsing and remitting", and then in a "progressive" form affecting more than 2.5 million people worldwide. The most common initial symptoms reported are: changes in sensation in the arms, legs or face, complete or partial vision loss, weakness, unsteadiness when walking, and balance problems.

Stroke and traumatic brain injury can also cause neuronal loss and lead to cognitive decline. Stroke can be classified into two major categories: ischemic and hemorrhagic. Ischemia is due to interruption of the blood supply, while hemorrhage is due to rupture of a blood vessel or an abnormal vascular structure. Stroke can cause permanent neurological damage, complications and death if not promptly diagnosed and treated. It is the third leading cause of death and the leading cause of adult disability in the United States and Europe.

At present, most treatments of these incurable neurological disorders are symptomatic. The discovery of the presence of neural stem/progenitor cells (NSCs) in the adult brain has opened new approaches for therapeutic interventions [Martino G et al., Nat Rev Neurosci. 2006, 7, 395]. NSCs are multipotent progenitor cells which can differentiate into all different nervous cell types (e.g., neurons, astrocytes and oligodendrocytes) and can contribute to neurogenesis in adulthood. Neurogenesis is known to persist throughout adulthood in two regions of the mammalian brain: the subventricular zone (SVZ) of the lateral ventricles and the dentate gyrus of the hippocampus. In these regions, NSCs continue to divide and give rise to new functional neurons and glial cells [Zhao C et al., Cell. 2008, 132, 645]. It has been shown that a variety of factors can stimulate adult hippocampal neurogenesis e.g., adrenalectomy, voluntary exercise, enriched environment, hippocampus dependent learning and anti-depressants.

The differentiation of these NSCs is regulated by key factors involved in neurogenesis and stem cell development. Hence, potent inducing agents for NSCs differentiation represent an innovative approach with good potential to treat or even cure neurological diseases by resupplying the central nervous system (CNS) with new functional nerve cells. Some proteic growth factors (e.g., fibroblast growth factor (FGF) and epidermal growth factor (EGF)) are able to induce the differentiation of NSCs into mature neural cells [Caldwell MA et al., Nat Biotechnol. 2001, 19, 475]. However, few non-proteic synthetic molecules have been shown to confer such effect. [Coowar D et al., J Med Chem. 2004, 47, 6270] [Warashina M et al., Angew Chem Int Ed. 2006, 45, 591] [Saxe JP et al., Chem Biol. 2007, 14, 1019].

Notch signaling controls a wide variety of cell fate decisions, including the differentiation of NSCs during neurogenesis [Artavanis-Tsakonas, S, Science. 1999, 284, 770]. Hence, compounds inhibiting the Notch signaling pathway would increase the production of neurons from NSCs. Inhibition of Notch signaling pathway represents a promising tool for treating different kinds of diseases and disorders, such as Alzheimer disease (Beher D et al., Exp Opin lnvestigat Drugs. 2005, 14, 1385); multiple sclerosis [John GR et al., Nat Med. 2002, 8, 1115]; brain tumours [Miele L et al., Curr Mol Med. 2006, 6, 905], and autoimmune disorders [Briend E et al., Curr Opin Mol Ther. 2005, 7, 56].

Although inflammation is not strictly a CNS disorder, it is frequently associated with brain injury, neurodegenerative diseases, and radiation treatment for brain tumors, which often causes deficits in cognition. Adult neurogenesis is also downregulated by endotoxin-induced inflammation and can be restored by anti-inflammatory treatments [Ekdahl CT et al., PNAS. 2003, 100, 13632]. During the development of neurodegenerative or demyelinating diseases, microglial cells, the brain resident monocyte-macrophage cell population, become highly activated [Klegeris A et al., Curr Opin Neurol. 2007, 20, 351]. This activation produces large amounts of devastating pro-inflammatory cytokines, like tumour necrosis factor-α (TNF-α), interleukin-1β (IL-1β) as well as free radicals like nitric oxide (NO) and superoxide anion. In AD, the extracellular depositions of amyloid beta (Aβ) represent the major histological lesion and are responsible for the death of neurons by a currently unclear mechanism. One theory involves neuroinflammation which is supported by studies showing clustering of microglial cells within Aβ depositions in human brain tissues. According to the neuroinflammatory hypothesis of neurodegenerative diseases, drugs with an anti-inflammatory mode of action should slow the disease progression.

The complex nature of the pathogenesis of neurodegenerative disease may require simultaneous use of several drugs directed at various factors contributing to the disease pathogenesis, including neuroinflammatory reactions. The urgent need for such combination therapies is being recognized due to the failure, or marginally protective effects, observed by using single anti-inflammatory and other neuroprotective agents. Hence, anti-inflammatory compounds promoting neurogenesis and/or the differentiation of oligodendrocyte precursors are excellent agents for the treatment of neurodegenerative or demyelinating diseases. Moreover, if these compounds modulate cell differentiation through inhibition of the Notch signaling pathway, they can be useful for the treatment of cancer where the Notch signaling is up-regulated.

Tocopherol [WO2005030748A1] and Resveratrol [WO2007099162A2] long-chain alcohol compounds are known to have these dual bioactivies, promoting the differentiation of neural progenitor cells and modulating neuroinflammation. Both types of compound are known to act through the Notch signaling pathway.

It has now been found that certain chromenone derivatives promote differentiation of neural progenitor cells and modulate neuroinflammation.

### BRIEF DESCRIPTION OF DRAWINGS

Figure 1 shows the results of an experiment to measure the ability of test compounds to down-regulate iNOS and TNF-α gene expression in microglial cells
Figure 2 shows the results of an experiment to investigate the ability of test compounds to inhibit the Notch signaling pathway, by measuring Hes5 gene expression and Mash1 transcripts in the treated cultures
Figure 3 shows the results of an experiment to measure the ability of test compounds to induce the differentiation of SH-SY5Y neuroblastoma cell line by modulating the gene expression of Hash1 and Notch1.

### DESCRIPTION OF THE INVENTION

In one aspect, the present invention provides a compound of the general formula (I) or (II) 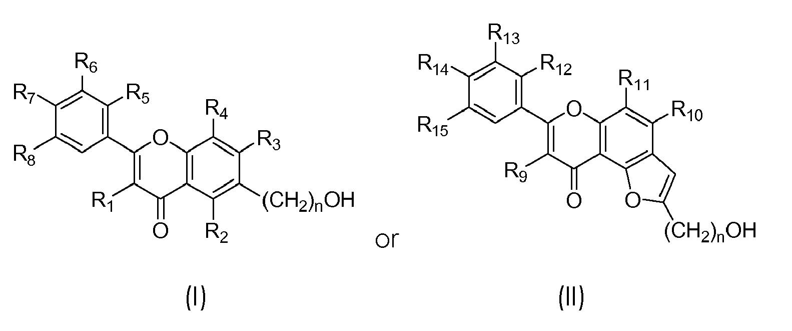 or a pharmaceutically acceptable salt thereof, wherein each of R₁, R₂, R₃, R₄, R₅, R₆, R₇, R₈, R₉, R₁₀, R₁₁, R₁₂, R₁₃, R₁₄ and R₁₅ independently represents a hydrogen atom, a hydroxyl group, a (C₁-C₆) alkyl group, a (C₂-C₆) alkenyl group, a (C₂-C₆) alkynyl group, a (C₁-C₆) alkoxy group, a hydroxy(C₁-C₆) alkyl group, a (C₁-C₆) alkoxy(C₁-C₆) alkyl group, a (C₁-C₆) alkanoyloxy group, a (C₁-C₆) alkanoyl group, a carboxyl group, a (C₁-C₆) alkoxycarbonyl group, a carbamoyl group, a (C₁-C₆) alkylaminocarbonyl group, a di(C₁-C₆) alkylaminocarbonyl group, a halogen atom, a halo(C₁-C₆) alkyl group, a halo(C₁-C₆) alkoxy group, a nitrile group, an amino group, a (C₁-C₆) alkylamino group, a di(C₁-C₆) alkylamino group or a (C₁-C₆) alkanoylamino group, or two adjacent groups represent a methylenedioxy group, and n is an integer in the range of from 8 to 25.

Compounds according to the present invention can modulate the cellular fate of neural stem cells and promote the differentiation of these neural precursors to functional neurons and glial cells. In addition, compounds according to the present invention are able to reduce the inflammatory component of neurological disorders by modulating the activation of microglia and/or by reducing reactive gliosis.

The compounds are useful for the treatment of neurodegenerative diseases such as Alzheimer's disease, demyelinating diseases such as multiple sclerosis, Parkinson's disease, stroke (ischemic and hemorrhagic), traumatic brain injury and cancers, such as brain cancer.

Without wishing to be bound by theory, it is believed that the biological activity of these compounds is related to the inhibition of the Notch signaling pathway. Accordingly, compounds according to the present invention are expected to be useful for the treatment of diseases associated with an up-regulated Notch signaling pathway activity, including cancer, in particular brain cancer.

In one embodiment, n in a compound of formula (I) is an integer in the range of from 10 to 20.

In another embodiment, n in a compound of formula (II) is an integer in the range of from 8 to 18.

Examples of particular values for n are 9, 10, 11, 12, 13 or 14 and 15.

In another embodiment, each of R₁, R₂, R₃, R₄, R₅, R₆, R₇, R₈, R₉, R₁₀, R₁₁, R₁₂, R₁₃, R₁₄ and R₁₅ independently represents a hydrogen atom, a hydroxyl group or a (C₁-C₆) alkoxy group. An example of a (C₁-C₆) alkoxy group is methoxy.

In one embodiment, each of R₄ and R₁₁ represents a hydrogen atom.

In yet another embodiment, each of R₇ and R₁₄ represents a hydroxy group or a (C₁-C₆) alkoxy group. An example of a (C₁-C₆) alkoxy group is a methoxy group.

In another embodiment, each of R₅ and R₁₂ represents a hydrogen atom, a hydroxyl group or a (C₁-C₆) alkoxy group. Examples of values for R₅ and R₁₂ are a hydrogen atom and a methoxy group.

In another embodiment, each of R₆ and R₁₃ represents a hydrogen atom, a hydroxyl group or a (C₁-C₆) alkoxy group. Examples of values for R₆ and R₁₃ are a hydrogen atom and a methoxy group.

In another embodiment, each of R₁ and R₉ represents a hydrogen atom, a hydroxy group or a (C₁-C₆) alkoxy group. For example, each of R₁ and R₉ may represent a hydrogen atom or a hydroxy group.

In another embodiment, each of R₂, R₃ and R₁₀ represents a hydrogen atom, a hydroxy group or a (C₁-C₆) alkoxy group. An example of a (C₁-C₆) alkoxy group is a methoxy group.

In another embodiment, each of R₈ and R₁₅ represents a hydrogen atom, a hydroxy group or a (C₁-C₆) alkoxy group. An example of a (C₁-C₆) alkoxy group is a methoxy group.

As used herein, the term (C₁-C₆) alkyl refers to an unbranched or branched alkyl group having from one to six carbon atoms. An example of a (C₁-C₆) alkyl group is methyl.

The term (C₂-C₆) alkenyl refers to an unbranched or branched alkenyl group having from two to six carbon atoms.

The term (C₂-C₆) alkynyl refers to an unbranched or branched alkynyl group having from two to six carbon atoms.

The term (C₁-C₆) alkoxy group refers to an unbranched or branched alkoxy group having from one to six carbon atoms. An example of a (C₁-C₆) alkoxy group is methoxy.

The term (C₁-C₆) alkanoyl group refers to an unbranched or branched alkanoyl group having from one to six carbon atoms. An example of a (C₁-C₆) alkanoyl group is ethanoyl.

The term halogen atom refers to fluorine, chlorine, bromine and iodine.

The terms halo(C₁-C₆) alkyl group and halo(C₁-C₆) alkoxy refer respectively to a (C₁-C₆) alkyl group and (C₁-C₆) alkoxy group in which one or more, for example one, two or three hydrogen atoms has been replaced with a halogen atom.

It will be appreciated that certain compounds of formula (I) and (II) may form pharmaceutically acceptable salts with pharmaceutically acceptable bases or acids. It will also be appreciated that certain compounds of formula (I) and (II) contain a chiral center and may therefore be prepared and isolated in a stereochemically pure form.

The compounds of the general Formula (I) can be obtained by methods such as the following:

### Process A: R₂ = H ; R₁ = OH

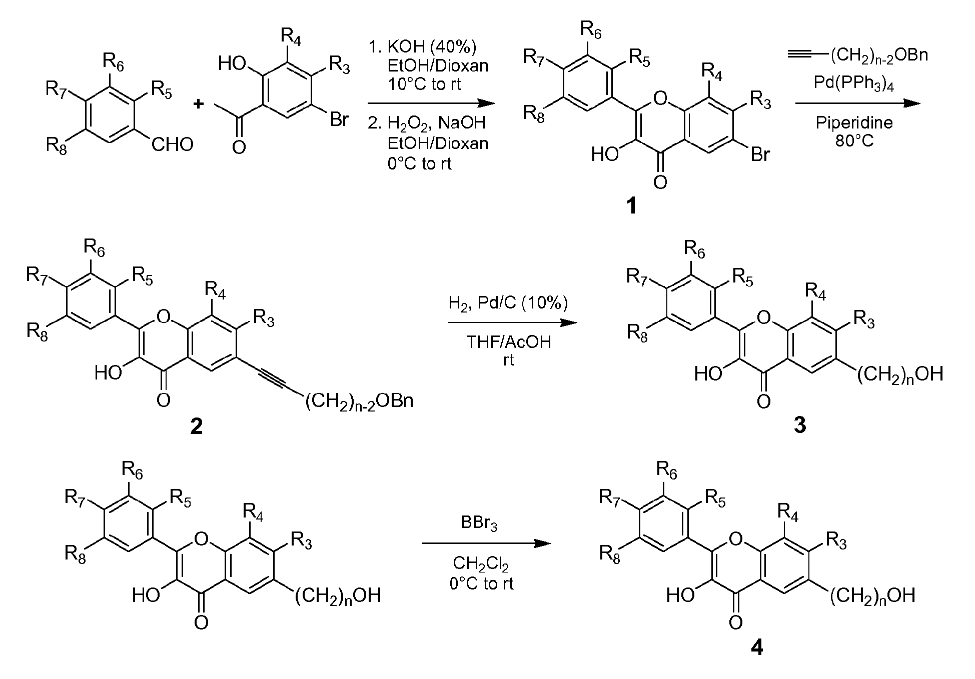

Compounds 4 of the present invention shown in Process A can be obtained by the following four steps. The flavonols 1 were synthesized from the 5' bromo-2'-hydrohyacetophenones. Aldol condensation with the commercial benzaldehydes gave the corresponding chalcones, and then Algar-Flynn-Oyamada (AFO) oxidation gave the bromoflavonols 1. The latter then underwent a Sonogashira reaction with the different alkynes in the presence of tetrakis(triphenyphosphine) palladium to give the corresponding compounds 2. Catalytic hydrogenation in the presence of palladium on charcoal afforded the substituted compounds 3. Compounds 3 substituted with methoxy groups were then demethylated using boron tribromide in dichloromethane to give the hydroxyl groups.

### Process B: R₁, R₂, R₃, R₆, R₇ = OH ; R₄, R₅, R₈ = H

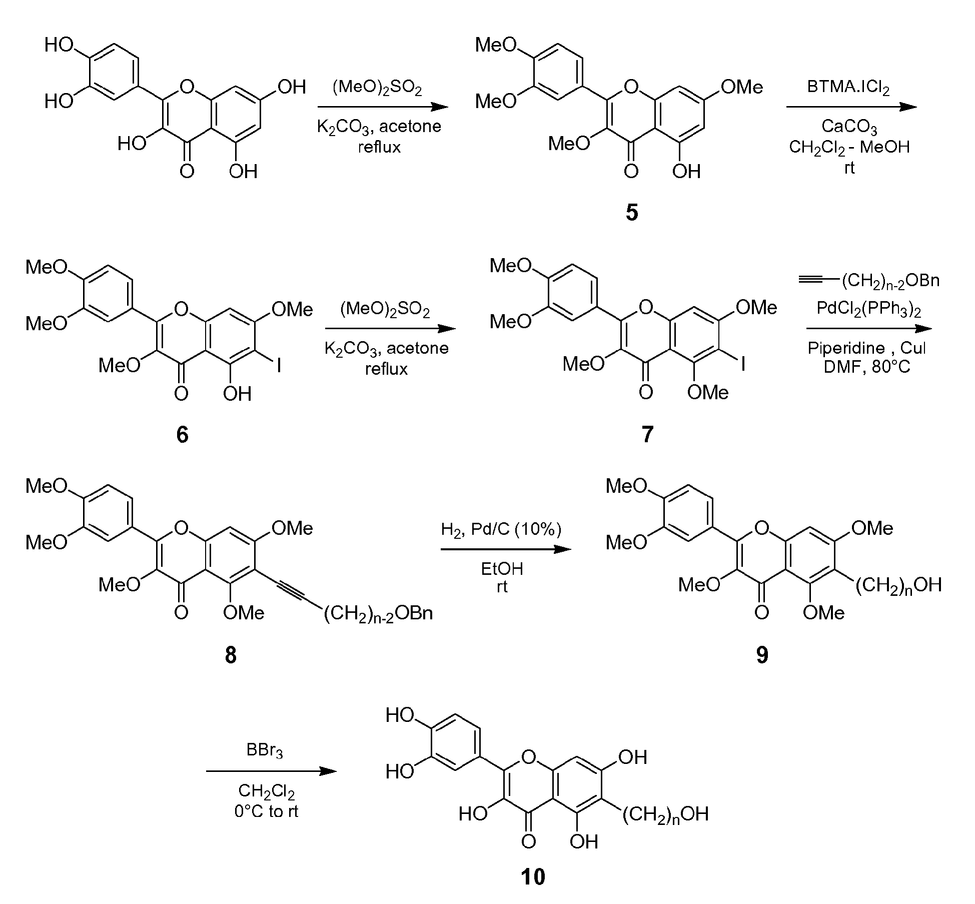

Compounds 10 of the present invention shown in Process B can be obtained by the following six steps. Compound 5 is obtained through tetramethylation of commercial Quercetin with dimethylsulfate. The compound 5 was then iodinated selectively on position 5 using benzyltrimethyl ammonium chloroiodate (BTMA.ICl₂) and methylated to give compound 7. The latter then underwent a Sonogashira reaction with the different alkynes to give the corresponding compounds 8. Catalytic hydrogenation in the presence of palladium on charcoal afforded the substituted compounds 9, which were then demethylated using boron tribromide in dichloromethane to give the free hydroxyl groups.

### Process C: R₂, R₃ = OH ; R₁, R₄ = H

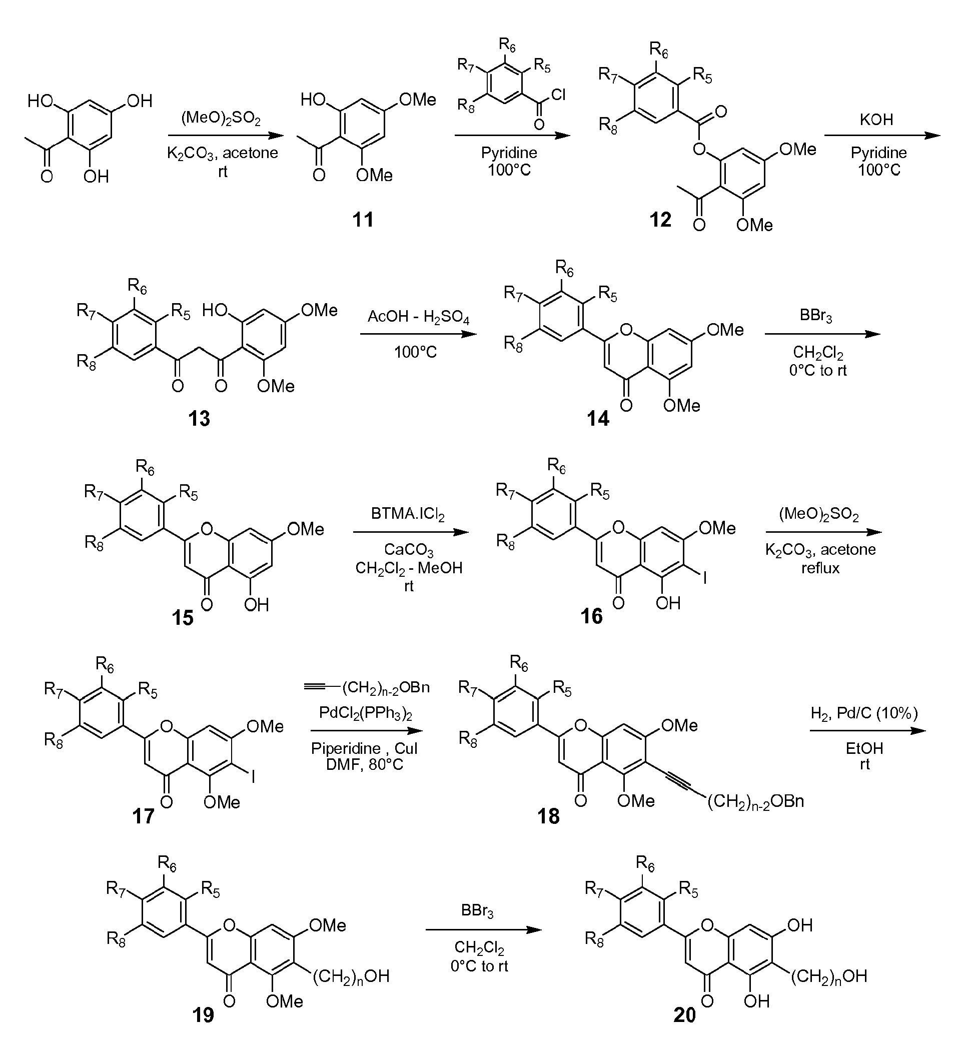

Flavones long chain ω-alkanols 20 of the present invention shown in Process C can be obtained by the following ten steps from commercial 2',4',6'-trihydroxyacetophenone. Following selective methylation of the latter compound, the flavones 14 were obtained in three steps using Baker-Vankataraman rearrangement. Selective demethylation with boron tribromide and iodination with benzyltrimethyl ammonium chloroiodate (BTMA.ICl₂) gave iodoflavones 17. These compounds then underwent a Sonogashira reaction with the different alkynes to give the corresponding compounds 18. Catalytic hydrogenation in the presence of palladium on charcoal afforded the substituted compounds 19, which were then demethylated using broron tribromide in dichloromethane to give the free-hydroxy flavones 20.

The compounds of the general Formula (II) can be obtained by methods such as the following:

### Process D: R₉, R₁₀, R₁₃, R₁₄ = OH ; R₁₁, R₁₂, R₁₅ = H

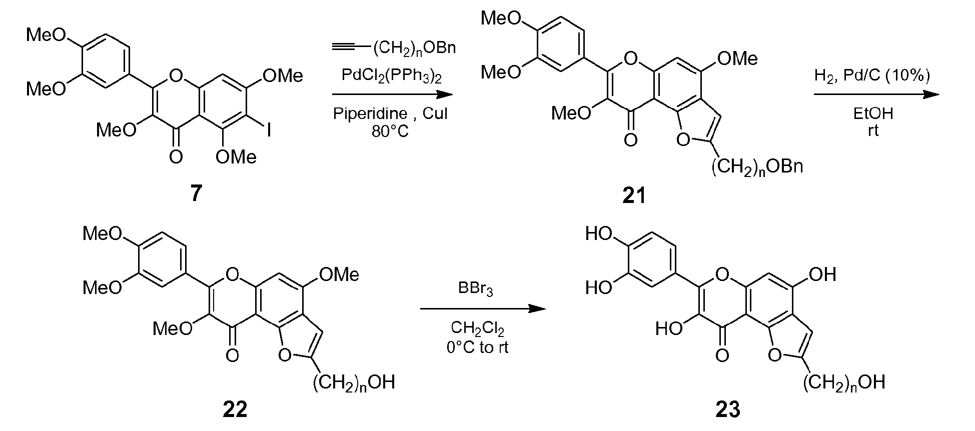

Furo[2,3-f]flavonol long chain ω-alkanols 23 of the present invention shown in Process D can be obtained in three steps from the iodomethylated quercetin 7. By modifying the Sonogashira reaction protocol i.e. using piperidine as solvent and base, we were able to devise a new single step method for the production of 9*H*-furo[2,3-f]benzopyran-9-one derivatives 21. These compounds then underwent catalytic hydrogenation and demethylation with boron tribromide to give compounds 23.

### Process E: R₁₀ = OH ; R₁₁ = H

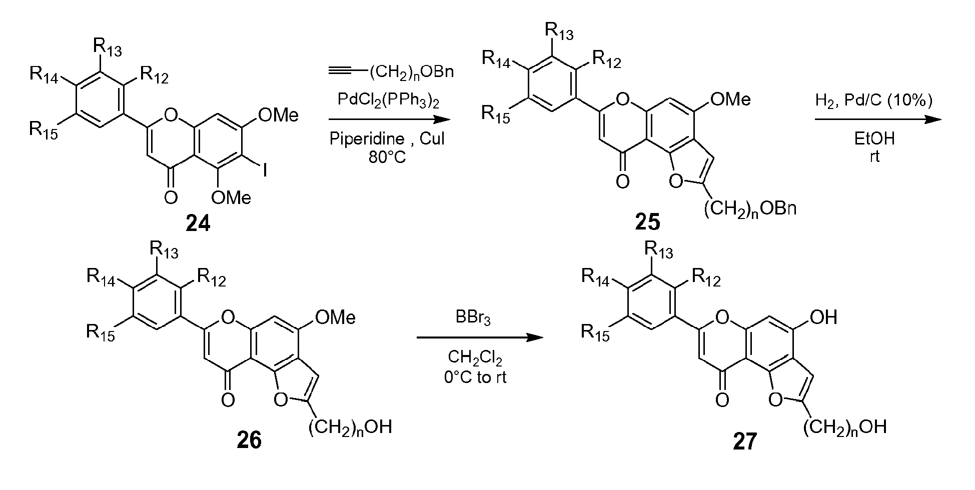

As in Process D, Furo[2,3-f]flavone long chain ω-alkanols 27 of the present invention shown in Process E can be obtained in three steps from the iodomethylated flavones 24 using the Sonogashira-cyclisation tandem reaction. The compounds 27 are then obtained following catalytic hydrogenation and demethylation with boron tribromide.

According to another aspect, therefore, the present invention provides a process for the preparation of a compound of general formula (I) or (II) or a pharmaceutically acceptable salt thereof as defined hereinabove, which comprises
a) for a compound of formula (II), reacting a compound of the general formula (III) 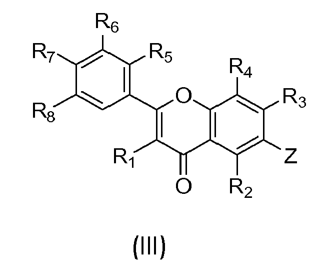 or a protected derivative thereof, in which Z represents a leaving atom or group (for example a halogen atom such as an iodine atom) with a compound of formula 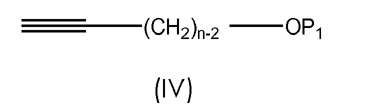 in which P₁ represents a hydrogen atom or a hydroxyl protecting group, in the presence of a Group VIII metal catalyst under cyclisation conditions (e.g. in the absence of a solvent);
b) for a compound of formula (I), reducing a compound of the general formula (V) 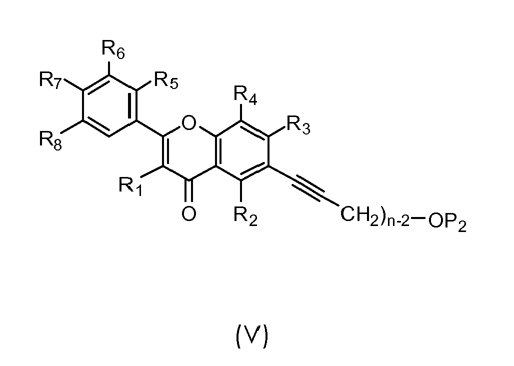 or a protected derivative thereof, in which P₂ represents a hydrogen atom or a hydroxyl protecting group;
   followed by removing any protecting groups and if desired, forming a pharmaceutically acceptable salt.

The starting materials of formulae (III) and (V) are believed to be novel, and accordingly are provided as further aspects of the invention.

Conveniently the compound and its pharmaceutically acceptable salts are administered to the patient in a pharmaceutical composition.

According to another aspect therefore, the present invention provides a pharmaceutical composition, which comprises a compound of formula (I) or (II), or a pharmaceutically acceptable salt thereof, and a pharmaceutically acceptable carrier.

The compounds may be formulated in any conventional manner, for example in a tablet, capsule, suppository, solution, syrup or powder, depending upon the intended route of administration.

The dose of the compound administered to the patient will depend upon many different factors to be considered by the attending physician, including the age, weight and sex of the patient, the route of administration and the nature of the condition being treated. In general, the compound will be administered at a dose equivalent to administering the compound in the range of from about 0.01 mg/kg to about 100 mg/kg body weight.

In another aspect, the present invention provides a compound of formula (I) or (II), or a pharmaceutically acceptable salt thereof, for use in therapy.

As described hereinabove, the compounds according to the present invention are useful in the treatment of neurodegenerative disease.

According to another aspect, therefore, the present invention provides a compound of formula (I) or (II), or a pharmaceutically acceptable salt thereof, for use in the treatment of a neurodegenerative disease.

In another aspect, the present invention provides a method of treating a neurodegenerative disease in a patient requiring treatment, which comprises administering an effective amount of a compound of formula (I) or (II), or a pharmaceutically acceptable salt thereof as defined hereinabove.

The compounds according to the present invention may be administered alone or co-administered with compounds working by a different mechanism, for example neuroprotectant agents. In one embodiment, the compounds are co-administered compounds with an acetylcholinesterase inhibitor (e.g. Aricept) for Alzheimer's disease or L-DOPA for Parkinson disease]

The following examples illustrate the invention.

### Example synthesis of compound 4 of general Fomula (I)

### Example 1: synthesis of 6-bromo-2-(3,4-dimethoxyphenyl)-3-hydroxy-4H-chromen-4-one (1a)

To a solution of 3,4-dimethoxybenzaldehyde (2.0 g, 9.30 mmol, 1eq) and 5'-bromo-2'-hydroxyacetophenone (1.55 g, 9.30 mmol, 1 eq) in dioxane (16 mL) and ethanol (23 mL), 40% (^{w}/ᵥ) KOH solution (6.6 mL, 46.50 mmol, 5 eq) was added dropwise at 10°C. The reaction mixture was stirred for 144 h at room temperature. 150 mL CH₂Cl₂ was added and the organic layer was washed with H₂O (3*50 mL), dried over magnesium sulphate and concentrated in vacuo.

The yellow solid was dissolved in dioxane (34 mL) and ethanol (86 mL) and a 5.4% (^{w}/ᵥ) NaOH solution (27.6, 37.50 mmol, 4 eq) was added dropwise. 30% H₂O₂ solution (3.6 mL, 31.75 mmol, 3.4 eq) was added dropwise. The reaction mixture was stirred on ice for 2h and subsequently for 24 h at room temperature, resulting in a yellow suspension. After acidification with 50 mL HCl (2M), the aqueous phase was extracted with AcOEt (3*50 mL). The combined organic layers were washed with water, dried over magnesium sulphate and concentrated in vacuo. The product was recrystallized from ethanol to give 1.25 g (36%) of a yellow solid.
Molecular Weight: 377.19 (C₁₇H₁₃BrO₅).

**¹H-NMR** δ (TFA-d, 300 MHz) ppm (*J* in Hz): 3.99 (s, 3H, MeO), 4.00 (s, 3H, MeO), 7.12 (d, 1H, *J*=8.7, H-5'), 7.63 (d, 1H, *J*=9.0, H-8), 7.94 (dd, 1H, *J*=9.0, 2.1, H-7), 8.02 (d, 1H, *J*=2.0, H-2'), 8.08 (dd, 1H, *J*=8.7, 2.0, H-6'), 8.37 (d, 1H, *J*=2.1, H-5).

**¹³C-NMR** δ (TFA-d, 75 MHz) ppm: 54.9, 55.2 (MeOx2), 111.2 (C-2'), 112.0 (C-5'), 116.3 (C-6), 119.2 (C-6'), 120.5 (C-1'), 121.1 (C-4a), 124.7 (C-8), 126.6 (C-5), 136.4 (C-3), 138.7 (C-7), 148.0 (C-8a), 152.6 (C-4'), 153.9 (C-3'), 155.8 (C-2), 170.1 (C-4).

### Example 2: synthesis of 2-(4-benzyloxy-3-methoxyphenyl)-6-bromo-3-hydroxy-4H-chromen-4-one (1b)

The compound 2-(4-benzyloxy-3-methoxyphenyl)-6-bromo-3-hydroxy-4*H-*chromen-4-one (1b) was prepared by the method of Example 1, but using 4-(benzyloxy)-3-methoxybenzaldehyde instead of 3,4-dimethoxy benzaldehyde.
**Molecular Weight:** 453.28 (C₂₃H₁₇BrO₅) .

**¹H-NMR** δ (CDCl₃, 300 MHz) ppm (*J* in Hz): 3.95 (s, 3H, MeO), 5.25 (s, 2H, C*H*₂Ph), 6.97 (d, 1H, *J*=8.6, H-5'), 7.37 (m, 7H, H-7,8, CH₂*Ph*), 7.73 (d, 1H, *J*=2.1, H-2'), 7.79 (dd, 1H, *J*=8.6, 2.1, H-6'), 8.32 (d, 1H, *J*=2.0, H-5).

**¹³C-NMR** δ (CDCl₃, 75 MHz) ppm: 55.4 (MeO), 70.0 (CH₂Ph), 110.5 (C-2'), 112.5 (C-5'), 117.0 (C-6), 119.3 (C-6'), 120.7 (C-8), 121.3 (C-1'), 122.8 (C-4a), 126.5 (CH₂*Ph*), 127.1, 127.3 (C-5, CH₂*Ph*), 127.9 (CH₂*Ph*), 135.6 (C-7), 135.7 (C-3), 137.1 (CH₂*Ph*), 144.8 (C-8a), 148.6 (C-4'), 149.4 (C-3'), 153.1 (C-2), 171.1 (C-4).

Following the method of Example 1, the following compounds shown in Process A are prepared:
6-Bromo-2-(3,4-dimethoxyphenyl)-3-hydroxy-7-methoxy-4*H*-chromen-4-one
2-(4-Benzyloxy-3-methoxyphenyl)-6-bromo-3-hydroxy-7-methoxy-4*H*-chromen-4-one
6-Bromo-2-(3,4-dimethoxyphenyl)-3-hydroxy-8-methoxy-4*H*-chromen-4-one
2-(4-Benzyloxy-3-methoxyphenyl)-6-bromo-3-hydroxy-8-methoxy-4*H*-chromen-4-one
6-Bromo-2-(3,4-dimethoxyphenyl)-3-hydroxy-7,8-dimethoxy-4*H*-chromen-4-one
2-(4-Benzyloxy-3-methoxyphenyl)-6-bromo-3-hydroxy-7,8-dimethoxy-4*H-*chromen-4-one

### Example 3: synthesis of 6-(12-(benzyloxy)dodec-1-ynyl)-2-(3,4-dimethoxyphenyl)-3-hydroxy-4H-chromen-4-one (2a)

To a solution of 6-bromo-2-(3,4-dimethoxyphenyl)-3-hydroxy-4*H*-chromen-4-one (0.50 g, 1.33 mmol, 1 eq) and tetrakis(triphenylphosphine)palladium (0.11 g, 0.09 mmol, 0.07 eq) in piperidine (2 mL) at 80°C, a solution of 1-((dodec-11-ynyloxy)methyl)benzene (0.54 g, 1.99 mmol, 1.5 eq) in piperidine (2 mL) was added. The reaction mixture was heated for 3 h at 80°C and then quenched with saturated ammonium chloride (50 mL). The aqueous phase was extracted with dichloromethane (3*50 mL). The combined organic layers were washed with brine, dried over magnesium sulfate and concentrated in vacuo. The residue was purified by silica gel flash chromatography (heptanes /ethyl acetate: 7/3) to give 0.68 g (90%) of a yellow solid.
**Molecular Weight:** 568.70 (C₃₆H₄₀O₆).

**¹H-NMR** δ (CDCl₃, 300 MHz) ppm (*J* in Hz): 1.31 (s large, 12H, H-5" to H-10"), 1.41 to 1.64 (m, 4H, H-4",11"), 2.43 (t, 2H, *J*=6.9, H-3"), 3.46 (t, 2H, *J*=6.6, H-12"), 3.97 (s, 3H, MeO), 3.99 (s, 3H, MeO), 4.50 (s, 2H, C*H*₂Ph), 6.99 (s large, 1H, OH), 7.01 (d, 1H, *J*=8.6, H-5'), 7.33 (m, 5H, CH₂*Ph*), 7.49 (d, 1H, *J*=8.8, H-8), 7.66 (dd, 1H, *J*=8.8, 2.1, H-7), 7.83 (d, 1H, *J*=2.0, H-2'), 7.87 (dd, 1H, *J*=8.6, 2.0, H-6'), 8.24 (d, 1H, *J*=2.1, H-5) .

**¹³C-NMR** δ (CDCl₃, 75 MHz) ppm: 19.4 (C-3"), 26.2 (C-10"), 28.6 to 29.8 (C-4" to C-9", C-11"), 56.0 (MeOx2), 70.5 (C-12"), 72.8 (CH₂Ph), 79.1 (C-1 "), 91.8 (C-2"), 110.7, 110.9 (C-2', C-5'), 118.2 (C-8), 120.5, 120.8 (C-6, C-1'), 121.5 (C-6'), 123.5 (C-4a), 127.4 (C-5), 127.6, 128.3 (CH₂*Ph*), 136.3 (C-7), 137.8 (C-3), 138.7 (CH₂*Ph*), 145.1 (C-8a), 148.9 (C-4'), 150.8 (C-3'), 154.2 (C-2), 172.5 (C-4).

### Example 4: synthesis of 2-(4-benzyloxy-3-methoxyphenyl)-6-(12-benzyloxydodec-1-ynyl)-3-hydroxy-4H-chromen-4-one (2b)

The compound 2-(4-benzyloxy-3-methoxyphenyl)-6-(12-benzyloxydodec-1-ynyl)-3-hydroxy-4*H*-chromen-4-one (2b) was prepared by the method of Example 3, but using 2-(4-benzyloxy-3-methoxyphenyl)-6-bromo-3-hydroxy-4*H-*chromen-4-one instead of 6-bromo-2-(3,4-dimethoxyphenyl)-3-hydroxy-4*H-*chromen-4-one.
**Molecular Weight:** 644.80 (C₄₂H₄₄O₆).

**¹H-NMR** δ (CDCl₃, 300 MHz) ppm (*J* in Hz): 1.26 (s large, 12H, H-5" to H-10"), 1.36 to 1.64 (m, 4H, H-4",11"), 2.43 (t, 2H, *J*=7.0, H-3"), 3.47 (t, 2H, *J*=6.7, H-12"), 4.00 (s, 3H, MeO), 4.50 (s, 2H, C*H*₂Ph), 5.26 (s, 2H, C*H*₂Ph), 6.96 (s large, 1H, OH), 7.02 (d, 1H, *J*=8.7, H-5'), 7.28 to 7.50 (m, 11H, H-8, CH₂*Ph*), 7.66 (dd, 1H, *J*=8.7, 2.0, H-7), 7.80 (dd, 1H, *J*=8.7, 2.0, H-6'), 7.85 (d, 1H, *J*=2.0, H-2'), 8.25 (d, 1H, *J*=2.0, H-5).

**¹³C-NMR** δ (CDCl₃, 75 MHz) ppm: 18.6 (C-3"), 25.4 (C-10"), 27.9 to 29.0 (C-4" to C-9", C-11"), 55.4 (MeO), 69.8 (C-12"), 70.0, 72.1 (CH₂Phx2), 79.1 (C-1 "), 91.1 (C-2"), 110.4 (C-2'), 112.5 (C-5'), 117.5 (C-8), 119.8, 120.1 (C-6, C-1'), 120.6 (C-6'), 123.2 (C-4a), 126.4 (C-5), 126.7, 126.8, 127.3, 127.5 (CH₂*Ph*), 135.5 (C-7), 137.0 (C-3), 137.8 (CH₂*Ph*), 144.5 (C-8a), 148.6 (C-4'), 149.5 (C-3'), 153.8 (C-2), 172.1 (C-4).

Following the method of Example 3, the following compounds shown in Process A are prepared:
6-(12-Benzyloxydodec-1-ynyl)-2-(3,4-dimethoxyphenyl)-3-hydroxy-7-methoxy-4*H*-chromen-4-one
6-(13-Benzyloxytridec-1-ynyl)-2-(3,4-dimethoxyphenyl)-3-hydroxy-7-methoxy-4*H*-chromen-4-one
2-(4-Benzyloxy-3-methoxyphenyl)-6-(12-benzyloxydodec-1-ynyl)-3-hydroxy-7-methoxy-4*H*-chromen-4-one
2-(4-Benzyloxy-3-methoxyphenyl)-6-(13-benzyloxytridec-1-ynyl)-3-hydroxy-7-methoxy-4*H*-chromen-4-one
6-(12-benzyloxydodec-1-ynyl)-2-(3,4-dimethoxyphenyl)-3-hydroxy-8-methoxy-4*H*-chromen-4-one
6-(13-benzyloxytridec-1-ynyl)-2-(3,4-dimethoxyphenyl)-3-hydroxy-8-methoxy-4*H*-chromen-4-one
2-(4-Benzyloxy-3-methoxyphenyl)-6-(12-benzyloxydodec-1-ynyl)-3-hydroxy-8-methoxy-4*H*-chromen-4-one
2-(4-Benzyloxy-3-methoxyphenyl)-6-(13-benzyloxytridec-1-ynyl)-3-hydroxy-8-methoxy-4*H*-chromen-4-one
6-(12-Benzyloxydodec-1-ynyl)-2-(3,4-dimethoxyphenyl)-3-hydroxy-7,8-dimethoxy-4*H*-chromen-4-one
6-(13-Benzyloxytridec-1-ynyl)-2-(3,4-dimethoxyphenyl)-3-hydroxy-7,8-dimethoxy-4*H*-chromen-4-one
2-(4-Benzyloxy-3-methoxyphenyl)-6-(12-benzyloxydodec-1-ynyl)-3-hydroxy-7,8-dimethoxy-4*H*-chromen-4-one
2-(4-Benzyloxy-3-methoxyphenyl)-6-(13-benzyloxytridec-1-ynyl)-3-hydroxy-7,8-dimethoxy-4*H*-chromen-4-one

### Example 5: synthesis of 2-(3,4-dimethoxyphenyl)-3-hydroxy-6-(12-hydroxydodecyl)-4H-chromen-4-one (3a)

To a solution of 6-(12-(benzyloxy)dodec-1-ynyl)-2-(3,4-dimethoxyphenyl)-3-hydroxy-4*H*-chromen-4-one (0.66 g, 1.16 mmol) in THF/acetic acid (9/1) (4 mL), was added 10% Pd/C (0.13 g, 20% ^{w}/_{w}). The reaction mixture was then stirred under hydrogen atmosphere for 5 h at room temperature. The solution was filtered through Celite and concentrated in vacuo. The residue was purified by silica gel flash chromatography (heptanes /ethyl acetate: 5/5) to give 0.49 g (87%) of a yellow solid.
**Molecular Weight:** 482.61 (C₂₉H₃₈O₆).

**¹H-NMR** δ (CDCl₃, 300 MHz) ppm (*J* in Hz): 1.26 (s large, 16H, H-3" to H-10"), 1.54 to 1.67 (m, 4H, H-2",11"), 2.73 (t, 2H, *J*=7.8, H-1 "), 3.64 (t, 2H, *J*=6.5, H-12"), 3.97 (s, 3H, MeO), 3.99 (s, 3H, MeO), 7.02 (d, 1H, *J*=8.6, H-5'), 7.51 (s, 2H, H-7, H-8), 7.85 (d, 1H, *J*=2.0, H-2'), 7.89 (dd, 1H, *J*=8.6, 2.0, H-6'), 8.02 (s, 1H, H-5).

**¹³C-NMR** δ (CDCl₃, 75 MHz) ppm: 25.7 (C-10"), 29.1 (C-3"), 29.4 to 29.7 (C-4" to C-9"), 31.3 (C-2"), 32.8 (C-11"), 35.3 (C-1 "), 56.0 (MeOx2), 63.1 (C-12"), 110.7, 110.9 (C-2', C-5'), 117.9 (C-8), 120.4 (C-1'), 121.4 (C-6'), 123.4 (C-4a), 123.9 (C-5), 134.3 (C-7), 137.7 (C-6), 139.5 (C-3), 145.0 (C-8a), 148.8 (C-4'), 150.7 (C-3'), 153.8(C-2), 173.2 (C-4).

### Example 6: synthesis of 3-hydroxy-2-(4-hydroxy-3-methoxyphenyl)-6-(12-hydroxydodecyl)-4H-chromen-4-one (3b)

The compound 3-hydroxy-2-(4-hydroxy-3-methoxyphenyl)-6-(12-hydroxydodecyl)-4*H*-chromen-4-one (3b) was prepared by the method of Example 5, but using 2-(4-benzyloxy-3-methoxyphenyl)-6-(12-benzyloxydodec-1-ynyl)-3-hydroxy-4*H*-chromen-4-one instead of 6-(12-(benzyloxy)dodec-1-ynyl)-2-(3,4-dimethoxyphenyl)-3-hydroxy-4*H*-chromen-4-one.
**Molecular Weight:** 468.58 (C₂₈H₃₆O₆).

**¹H-NMR** δ (CDCl₃, 300 MHz) ppm (*J* in Hz): 1.26 (s large, 16H, H-3" to H-10"), 1.52 to 1.70 (m, 4H, H-2",11"), 2.73 (t, 2H, *J*=7.4, H-1"), 3.64 (t, 2H, *J*=6.6, H-12"), 4.00 (s, 3H, MeO), 7.07 (d, 1H, *J*=8.6, H-5'), 7.51 (m, 2H, H-7, H-8), 7.82 (dd, 1H, *J*=8.6, 2.0, H-6'), 7.87 (d, 1H, *J*=2.0, H-2'), 8.02 (s, 1H, H-5).

**¹³C-NMR** δ (CDCl₃, 75 MHz) ppm: 25.7 (C-10"), 29.1 (C-3"), 29.4 to 29.6 (C-4" to C-9"), 31.3 (C-2"), 32.8 (C-11"), 35.3 (C-1 "), 56.1 (MeO), 63.1 (C-12"), 110.4 (C-2'), 114.6 (C-5'), 117.9 (C-8), 120.4 (C-1'), 121.8 (C-6'), 123.4 (C-4a), 123.9 (C-5), 134.3 (C-7), 137.6 (C-6), 139.5 (C-3), 145.2 (C-8a), 146.4 (C-4'), 147.6 (C-3'), 153.7 (C-2), 173.3 (C-4).

Following the method of Example 5, the following compounds shown in Process A are prepared:
2-(3,4-Dimethoxyphenyl)-3-hydroxy-6-(12-hydroxydodecyl)-7-methoxy-4*H-*chromen-4-one
2-(3,4-Dimethoxyphenyl)-3-hydroxy-6-(13-hydroxytridecyl)-7-methoxy-4*H-*chromen-4-one
3-Hydroxy-2-(4-hydroxy-3-methoxyphenyl)-6-(12-hydroxydodecyl)-7-methoxy-4*H*-chromen-4-one
3-Hydroxy-2-(4-hydroxy-3-methoxyphenyl)-6-(13-hydroxytridecyl)-7-methoxy-4*H*-chromen-4-one
2-(3,4-Dimethoxyphenyl)-3-hydroxy-6-(12-hydroxydodecyl)-8-methoxy-4*H-*chromen-4-one
2-(3,4-Dimethoxyphenyl)-3-hydroxy-6-(13-hydroxytridecyl)-8-methoxy-4*H-*chromen-4-one
3-Hydroxy-2-(4-hydroxy-3-methoxyphenyl)-6-(12-hydroxydodecyl)-8-methoxy-4*H*-chromen-4-one
3-Hydroxy-2-(4-hydroxy-3-methoxyphenyl)-6-(13-hydroxytridecyl)-8-methoxy-4*H*-chromen-4-one
2-(3,4-Dimethoxyphenyl)-3-hydroxy-6-(12-hydroxydodecyl)-7,8-dimethoxy-4*H-*chromen-4-one
2-(3,4-Dimethoxyphenyl)-3-hydroxy-6-(13-hydroxytridecyl)-7,8-dimethoxy-4*H-*chromen-4-one
3-Hydroxy-2-(4-hydroxy-3-methoxyphenyl)-6-(12-hydroxydodecyl)-7,8-dimethoxy-4*H*-chromen-4-one
3-Hydroxy-2-(4-hydroxy-3-methoxyphenyl)-6-(13-hydroxytridecyl)-7,8-dimethoxy-4*H*-chromen-4-one

### Example 7: synthesis of 2-(3,4-dihydroxyphenyl)-3-hydroxy-6-(12-hydroxydodecyl)-4H-chromen-4-one (4)

To a solution of 2-(3,4-dimethoxyphenyl)-3-hydroxy-6-(12-hydroxydodecyl)-4*H-*chromen-4-one (0.20 g, 0.41 mmol, 1 eq) in dichloromethane (10 mL) at 0°C, was added boron tribromide in dichloromethane (1.0 M, 2.5 mL, 2.49 mmol, 6 eq). The mixture was warmed to room temperature and then stirred for 22 h. The reaction mixture was then cooled to 0°C and methanol (10 mL) was added. The reaction mixture was heated to reflux for 2 h, and then concentrated in vacuo to give a yellow solid. Water (50 mL) was added and the aqueous phase was extracted with ethyl acetate (3*50 mL). The combined organic layers were washed with brine, dried over magnesium sulfate and concentrated in vacuo. The residue was recrystallized from ethanol to give 0.10 g (55%) of a yellow solid.
**Molecular Weight:** 454.56 (C₂₇H₃₄O₆).

**¹H-NMR** δ (DMSO-d6, 300 MHz) ppm (*J* in Hz): 1.22 (s large, 16H, H-3" to H-10"), 1.58 to 1.80 (m, 4H, H-2",11"), 2.70 (t, 2H, *J*=7.5, H-1"), 3.37 (t, 2H, *J*=6.8, H-12"), 6.89 (d, 1H, *J*=8.6, H-5'), 7.59 (dd, 1H, *J*=8.6, 2.1, H-6'), 7.61 (s, 2H, H-7, H-8), 7.74 (d, 1H, *J*=2.1, H-2'), 7.86 (s, 1H, H-5), 9.23, 9.28, 9.55 (3s, OHx3).

**¹³C-NMR** δ (DMSO-d6, 75 MHz) ppm: 28.0 (C-10"), 28.6 (C-3"), 29.0 to 29.4 (C-4" to C-9"), 31.3 (C-2"), 32.7 (C-11"), 35.6 (C-1"), 115.7, 116.0 (C-2', C-5'), 118.5 (C-8), 120.4 (C-6'), 121.5 (C-1'), 122.8 (C-4a), 123.6 (C-5), 134.4 (C-7), 138.3 (C-6), 139.1 (C-3), 145.5 (C-4'), 146.4 (C-8a), 148.0 (C-3'), 153.3 (C-2), 172.8 (C-4).

Following the method of Example 7, the following compounds shown in Process A are prepared:
2-(3,4-Dihydroxyphenyl)-3,7-dihydroxy-6-(12-hydroxydodecyl)-4*H*-chromen-4-one
2-(3,4-Dihydroxyphenyl)-3,7-dihydroxy-6-(13-hydroxytridecyl)-4*H*-chromen-4-one
2-(3,4-Dihydroxyphenyl)-3,8-dihydroxy-6-(12-hydroxydodecyl)-4*H*-chromen-4-one
2-(3,4-Dihydroxyphenyl)-3,8-dihydroxy-6-(13-hydroxytridecyl)-4*H*-chromen-4-one
2-(3,4-Dihydroxyphenyl)-3,7,8-trihydroxy-6-(12-hydroxydodecyl)-4*H*-chromen-4-one
2-(3,4-Dihydroxyphenyl)-3,7,8-trihydroxy-6-(13-hydroxytridecyl)-4*H*-chromen-4-one

### Example synthesis of compound 10 of general Formula (I)

### Example 8: synthesis of 2-(3,4-dimethoxyphenyl)-5-hydroxy-3,7-dimethoxy-4H-chromen-4-one (5)

To a solution of commercial quercetin dihydrate (5.0 g, 14.78 mmol, 1 eq) in acetone (200 mL), potassium carbonate (8.4 g, 60.60 mmol, 4.1 eq) and dimethylsulfate (5.61 mL, 59.12 mmol, 4 eq) were added under argon at 0°C. After vigorous stirring at reflux for 3h, the resulting mixture was filtered and the filtrate concentrated in vacuo. The residue was recristallized from water /ethanol (1/1) (150 mL) then washed with diethyl ether (50 mL) to afford 3.23 g (61%) of a pale yellow solid.
**Molecular Weight:** 358.34 (C₁₉H₁₈O₇).

**¹H-NMR** δ (CDCl₃, 300 MHz) ppm (*J* in Hz): 3.86 (s, 3H, MeO), 3.87 (s, 3H, MeO), 3.96 (s, 3H, MeO), 3.97 (s, 3H, MeO), 6.35 (d, 1H, *J*=2.1, H-6), 6.44 (d, 1H, *J*=2.1, H-8), 6.98 (d, 1H, *J*=8.7, H-5'), 7.68 (d, 1H, *J*=2.1, H-2'), 7.73 (dd, 1H, *J*=8.7, 2.1, H-6').

### Example 9: synthesis of 2-(3,4-dimethoxyphenyl)-5-hydroxy-6-iodo-3,7-dimethoxy-4H-chromen-4-one (6)

To a solution of 2-(3,4-dimethoxyphenyl)-5-hydroxy-3,7-dimethoxy-4*H-*chromen-4-one (6.05 g, 16.88 mmol, 1 eq) in dichloromethane-MeOH (5/2) (210 mL), calcium carbonate (11.8 g, 118.18 mmol, 7 eq) and benzyltrimethylammonium chloroiodate (BTMA-ICl₂) (5.88 g, 16.88 mmol, 1 eq) were added under argon at 0°C. After vigorous stirring at room temperature overnight, the resulting mixture was quenched with distillated water (150 mL), and the aqueous phase extracted with dichloromethane (3*150 mL). The combined organic layers were washed with brine, dried over magnesium sulfate and concentrated in vacuo. The residue was purified by silica gel column chromatography (heptanes /ethyl acetate: 7/3, 1/1 then 0/1) to give 4.93 g (60%) of a yellow solid.
**Molecular Weight:** 484.24 (C₁₉H₁₇IO₇).

**¹H-NMR** δ (CDCl₃, 300 MHz) ppm (*J* in Hz): 3.86 (s, 3H, MeO), 3.97 (s, 6H, MeOx2), 3.99 (s, 3H, MeO), 6.49 (s, 1H, H-8), 6.98 (d, 1H, *J*=8.4, H-5'), 7.68 (d, 1H, *J*=2.1, H-2'), 7.73 (dd, 1H, *J*=8.4, 2.1, H-6'), 13.63 (s, 1H, OH).

**¹³C-NMR** δ (CDCl₃, 75 MHz) ppm: 56.2 (MeO), 56.3 (MeO), 57.1 (MeO), 60.4 (MeO), 69.6 (C-6), 90.4 (C-8), 106.2 (C-4a), 111.0, 111.4 (C-2', C-5'), 122.4 (C-6'), 122.7 (C-1'), 139.1 (C-3), 148.9 (C-4'), 151.7 (C-3'), 156.3 (C-2), 157.2 (C-8a), 161.0 (C-5), 163.3 (C-7), 178.0 (C-4).

### Example 10: synthesis of 2-(3,4-dimethoxyphenyl)-6-iodo-3,5,7-trimethoxy-4H-chromen-4-one (7)

To a solution of 2-(3,4-dimethoxyphenyl)-5-hydroxy-6-iodo-3,7-dimethoxy-4*H-*chromen-4-one (1.0 g, 2.07 mmol, 1 eq) in THF (50 mL), potassium carbonate (0.56 g, 4.13 mmol, 2 eq) and dimethylsulfate (0.20 mL, 2.07 mmol, 1 eq) were added under argon at 0°C. After vigorous stirring at reflux overnight, the resulting mixture was quenched with distillated water (100 mL), and the aqueous phase was extracted with dichloromethane (3*100 mL). The combined organic layers were washed with brine, dried over magnesium sulfate and concentrated in vacuo. The residue was purified by silica gel column chromatography (heptanes /ethyl acetate: 7/3, 1/1 then 0/1) to give 0.68 g (66%) of a white solid.
**Molecular Weight:** 498.27 (C₂₀H₁₉IO₇).

**¹H-NMR** δ (CDCl₃, 300 MHz) ppm (*J* in Hz): 3.86 (s, 3H, MeO), 3.97 (s, 9H, MeOx3), 4.00 (s, 3H, MeO), 6.73 (s, 1H, H-8), 6.98 (d, 1H, *J*=8.7, H-5'), 7.70 (d, 1H, *J*=2.1, H-2'), 7.71 (dd, 1H, *J*=8.7, 2.1, H-6').

**¹³C-NMR** δ (CDCl₃, 75 MHz) ppm: 56.1 (MeO), 56.2 (MeO), 57.1 (MeO), 60.1 (MeO), 62.0 (MeO), 82.8 (C-6), 95.9 (C-8), 109.1 (C-4a), 111.0, 111.4 (C-2', C-5'), 121.9 (C-6'), 123.1 (C-1'), 141.1 (C-3), 148.5 (C-4'), 151.2 (C-3'), 153.6 (C-2), 158.8 (C-8a), 160.4 (C-5), 161.9 (C-7), 172.6 (C-4).

### Example 11: synthesis of 6-(12-(benzyloxy)dodec-1-ynyl)-2-(3,4-dimethoxyphenyl)-3,5,7-trimethoxy-4H-chromen-4-one (8)

To a solution of 2-(3,4-dimethoxyphenyl)-6-iodo-3,5,7-trimethoxy-4*H*-chromen-4-one (0.25 g, 0.50 mmol, 1 eq) in DMF (5 mL), piperidine (0.1 mL, 1 mmol, 2 eq), PdCl₂(PPh₃)₂ (0.025 g, 0.035 mmol, 0.07 eq), Cul (6.7 mg, 0.035 mmol, 0.07 eq) and 1-((dodec-11-ynyloxy)methyl)benzene (0.21 g, 0.75 mmol, 1.5 eq) were added under argon at room temperature. The reaction was stirred at 80°C overnight. The resulting mixture quenched with 50 g of chilled water and the aqueous phase was extracted with ethyl acetate (3*50 mL). The combined organic layers were washed with brine, dried over magnesium sulfate and concentrated in vacuo. The residue was purified by silica gel column chromatography (heptanes /ethyl acetate: 8/2, 7/3 then 1/1) to afford 0.21 g (66%) of a fatty colourless oil.
**Molecular Weight:** 642.78 (C₃₉H₄₆O₈).

**¹H-NMR** δ (CDCl₃, 300 MHz) ppm (*J* in Hz): 1.31 (s large, 12H, H-5" to H-10"), 1.41 to 1.70 (m, 4H, H-4",11"), 2.53 (t, 2H, *J*=6.9, H-3"), 3.46 (t, 2H, *J*=6.6, H-12"), 3.87 (s, 3H, MeO), 3.97 (s, 9H, MeOx3), 4.04 (s, 3H, MeO), 4.50 (s, 2H, C*H*₂Ph), 6.68 (s, 1H, H-8), 6.98 (d, 1H, *J*=9.0, H-5'), 7.33 (m, 5H, CH₂*Ph*), 7.70 (d, 1H, *J*=2.1, H-2'), 7.71 (dd, 1H, *J*=9.0, 2.1, H-6').

**¹³C-NMR** δ (CDCl₃, 75 MHz) ppm: 20.1 (C-3"), 26.3 (C-10"), 28.9 to 29. 9 (C-4" to C-9", C-11 "), 56.1 (MeO), 56.3 (MeO), 56.6 (MeO), 60.0 (MeO), 61.9 (MeO), 70.6 (C-12"), 71.2 (C-1 "), 73.0 (CH₂Ph), 95.2 (C-8), 100.1 (C-2"), 106.7 (C-4a), 110.9, 111.3 (C-2', C-5'), 113.1 (C-6), 121.8 (C-6'), 123.3 (C-1'), 127.5 (CH₂*Ph*), 127.7 (CH₂*Ph*), 128.4 (CH₂*Ph*), 138.8 (CH₂*Ph*), 141.2 (C-3), 148.8 (C-4'), 151.1 (C-3'), 153.3 (C-2), 157.1 (C-8a), 162.6 (C-5), 163.6 (C-7), 173.1 (C-4).

### Example 12: synthesis of 6-(12-hydroxydodec-1-yl)-2-(3,4-dimethoxyphenyl)-3,5,7-trimethoxy-4H-chromen-4-one (9)

To a solution of 6-(12-(benzyloxy)dodec-1-ynyl)-2-(3,4-dimethoxyphenyl)-3,5,7-trimethoxy-4*H*-chromen-4-one (0.20 g, 0.31 mmol, 1 eq) in EtOH / acetone (1/1) (6 mL), was added 10% Pd/C wet Degussa™ (0.15 g, 38% ^{w}/_{w}). The reaction mixture was then stirred under hydrogen atmosphere for 36 h at room temperature. The solution was filtered through Celite and concentrated in vacuo. The residue was purified by silica gel column chromatography (ethyl acetate / heptanes: 1/1 then 4/1) to afford 0.09 g (52%) of a white solid.
**Molecular Weight:** 556.69 (C₃₂H₄₄O₈).

**¹H-NMR** δ (CDCl₃, 300 MHz) ppm (*J* in Hz): 1.25 (s large, 16H, H-3" to H-10"), 1.51 to 1.60 (m, 4H, H-2",11"), 2.66 (t, 2H, *J*=8.1, H-1"), 3.63 (t, 2H, *J*=6.6, H-12"), 3.85 (s, 3H, MeO), 3.91 (s, 3H, MeO), 3.92 (s, 3H, MeO), 3.96 (s, 6H, MeOx2), 6.69 (s, 1H, H-8), 6.98 (d, 1H, *J*=9.0, H-5'), 7.71 (m, 2H, H- 2', H-6').

**¹³C-NMR** δ (CDCl₃, 75 MHz) ppm: 23.3 (C-1 "), 25.9 (C-10"), 29.6 to 30.0 (C-2" to C-9"), 32.9 (C-11"), 56.0 (MeO), 56.1 (MeO), 56.2 (MeO), 60.1(MeO), 62.6 (MeO), 63.2 (C-12"), 95.0 (C-8), 110.9, 111.4 (C-2', C-5'), 112.6 (C-4a), 121.8 (C-6'), 123.2 (C-6), 123.6 (C-1'), 141.2 (C-3), 148.8 (C-4'), 150.9 (C-3'), 153.2 (C-2), 156.7 (C-8a), 157.7 (C-5), 162.4 (C-7), 173.63 (C-4).

### Example 13: synthesis of 2-(3,4-dihydroxyphenyl)-3,5-dihydroxy-6-(12-hydroxydodecyl) -7-methoxy-4H-chromen-4-one (10)

To a solution of 6-(12-hydroxydodec-1-yl)-2-(3,4-dimethoxyphenyl)-3,5,7-trimethoxy-4*H*-chromen-4-one (0.05 g, 0.08 mmol, 1 eq) in dichloromethane (5 mL) at 0°C, was added boron tribromide in dichloromethane (1.0 M, 1.3 mL, 1.27 mmol, 15 eq). The mixture was warmed to room temperature and then stirred for 22 h. The reaction mixture was then cooled to 0°C and methanol (5 mL) was added. The reaction mixture was heated to reflux for 2 h, and then concentrated in vacuo to give a yellow solid. Water (50 mL) was added and the aqueous phase was extracted with ethyl acetate (3*50 mL). The combined organic layers were washed with brine, dried over magnesium sulfate and concentrated in vacuo. The residue was precipitated from aqueous ethanol to give 0.02 g (49%) of a pale-yellow solid.
**Molecular Weight:** 500.58 (C₂₈H₃₆O₈).

**¹H-NMR** δ (acetone-d6, 300 MHz) ppm (*J* in Hz): 1.29 (s large, 16H, H-3" to H-10"), 1.66 to 1.83 (m, 4H, H-2",11"), 2.65 (t, 2H, *J*=7.2, H-1"), 3.47 (t, 2H, *J*=6.9, H-12"), 3.89 (s, 3H, MeO), 6.76 (s, 1H, H-8), 6.99 (d, 1H, *J*=8.7, H-5'), 7.71 (dd, 1H, *J*=8.4, *J*=2.1, H-6'), 7.85 (d, 1H, *J*=2.1, H-2').

**¹³C-NMR** δ (acetone-d6, 75 MHz) ppm: 23.7 (C-1 "), 30.5 (C-10"), 30.8 to 31.6 (C-3" to C-9"), 34.6 (C-2"), 35.8 (C-11 "), 57.6 (MeO), 91.7 (C-8), 114.4 (C-6), 116.7, 117.2 (C-2', C-5'), 122.4 (C-6'), 124.8 (C-1'), 138.9 (C-3), 146.7 (C-4'), 149.2 (C-3'), 157.0 (C-5), 158.9 (C-2), 165.6 (C-7).

Following the method of Example 13, the following compounds shown in Process B are prepared:
2-(3,4-Dihydroxyphenyl)-3,5-dihydroxy-6-(13-hydroxytridecyl)-7-methoxy-4H-chromen-4-one
2-(3,4-Dihydroxyphenyl)-3,5,7-trihydroxy-6-(12-hydroxydodecyl)-4*H*-chromen-4-one
2-(3,4-Dihydroxyphenyl)-3,5,7-trihydroxy-6-(13-hydroxytridecyl)-4*H*-chromen-4-one

### Example synthesis of compound 20 of general Formula (I)

### Example 14: synthesis of 1-(2-hydroxy-4,6-dimethoxyphenyl)ethanone (11)

To a solution of commercial 2',4',6'-trihydroxyacetophenone dihydrate (15.0 g, 80.57 mmol, 1eq) in acetone (170 mL), potassium carbonate (23.4 g, 169.20 mmol, 2.1 eq) and dimethylsulfate (15.6 mL, 165.17 mmol, 2.05 eq) were added under argon at 0°C. After vigorous stirring at room temperature for 17h, the resulting mixture was filtered and the filtrate concentrated in vacuo. The resulting product **5** was recristallized from ethyl acetate / heptanes (3/97) to give 15.80 g (99%) of a pale yellow solid.
**Molecular Weight:** 196.20 (C₁₀H₁₂O₄).

**¹H-NMR** δ (CDCl₃, 300 MHz) ppm (*J* in Hz): 2.61 (s, 3H, C*H*₃CO), 3.82 (s, 3H, MeO), 3.85 (s, 3H, MeO), 5.91 (d, 1H, *J*=2.4, H- 3'), 6.05 (d, 1H, *J*=2.4, H-5'), 14.03 (s, 1H, OH).

### Example 15: synthesis of 2-acetyl-3,5-dimethoxyphenyl 3,4-dimethoxybenzoate (12)

3,4-dimethoxybenzoyl chloride (10.55 g, 52.72 mmol, 1.2 eq) was added to a suspension of 1-(2-hydroxy-4,6-dimethoxyphenyl)ethanone (8.62 g, 43.93 mmol, 1eq) in pyridine (120 mL). The reaction mixture was then stirred at 100°C for 10 mins. After cooling, to this suspension was added ethanol (100 mL) then water (100 mL). This solution was placed at 0°C and following scratching with a glass rod to afford white tiny crystals. The precipitate was filtered, washed with ethanol/water (1/1) (30 mL), dried to give 11.57 g (73%) of a white solid.
**Molecular Weight:** 360.36 (C₁₉H₂₀O₇).

**¹H-NMR** δ (CDCl₃, 300 MHz) ppm (*J* in Hz): 2.47 (s, 3H, C*H*₃CO), 3.82 (s, 3H, MeO), 3.85 (s, 3H, MeO), 3.94 (s, 3H, MeO), 3.95 (s, 3H, MeO), 6.37 (d, 1H, *J*=2.2, H-3'), 6.39 (d, 1H, *J*=2.2, H-5'), 6.92 (d, 1H, *J*=8.5, H-5"), 7.61 (d, 1H, *J*=2.0, H-2"), 7.79 (dd, 1H, *J*=2.0, *J*=8.5, H-6").

Following the method of Example 15, the following compounds shown in Process C are prepared:
2-Acetyl-3,5-dimethoxyphenyl 4-methoxybenzoate
2-Acetyl-3,5-dimethoxyphenyl 4-(benzyloxy)-3-methoxybenzoate
2-acetyl-3,5-dimethoxyphenyl 2,4,5-trimethoxybenzoate
2-Acetyl-3,5-dimethoxyphenyl 3,4,5-trimethoxybenzoate

### Example 16: synthesis of 1-(3,4-dimethoxyphenyl)-3-(2-hydroxy-4,6-dimethoxyphenyl) propane-1,3-dione (13)

To a suspension of 2-acetyl-3,5-dimethoxyphenyl 3,4-dimethoxybenzoate (10.31 g, 28.61 mmol, 1 eq) in pyridine (50 mL) was added crush hydroxide potassium (4.01 g, 71.52 mmol, 2.5 eq). The reaction mixture was stirred at 100°C for 10 mins. After cooling, this solution was poured into glacial acetic acid (30 mL) then ethanol (100 mL) and water (100 mL) was added. This solution was placed at 0°C and following scratching with a glass rod to afford yellow tiny crystals. The precipitate was filtered, washed with ethanol/water (1/1) (30 mL), dried to give 7.47 g (72%) of a pale yellow solid.
**Molecular Weight:** 360.36 (C₁₉H₂₀O₇).

**¹H-NMR** δ (CDCl₃, 300 MHz) ppm (*J* in Hz): ratio enol / 1,3-diketone: (52/48); *1*,*3-diketone:* 3.48 (s, 2H, COC*H*₂CO), 3.79 (s, 3H, MeO), 3.92 (s, 3H, MeO), 3.95 (s, 6H, MeOx2), 5.82 (d, 1H, *J*=2.4, H-3'), 6.08 (d, 1H, *J*=2.4, H-5'), 6.89 (d, 1H, *J*=8.1, H-5"), 7.53 (d, 1H, *J*=2.1, H-2"), 7.55 (dd, 1H, *J*=8.1, *J*=2.1, H-6"); enol : 3.80 (s, 3H, MeO), 3.90 (s, 3H, MeO), 3.92 (s, 3H, MeO), 3.95 (s, 3H, MeO), 4.50 (s, 1H, HC=C), 5.97 (d, 1H, *J*=2.4, H-3'), 6.09 (d, 1H, *J*=2.4, H-5'), 6.89 (d, 1H, *J*=8.1, H-5"), 7.43 (d, 1H, *J*=2.1, H-2"), 7.50 (dd, 1H, *J*=8.1, *J*=2.1, H-6").

Following the method of Example 16, the following compounds shown in Process C are prepared:
1-(2-Hydroxy-4,6-dimethoxyphenyl)-3-(4-methoxyphenyl)propane-1,3-dione
1-(4-Benzyloxy-3-methoxyphenyl)-3-(2-hydroxy-4,6-dimethoxyphenyl) propane-1,3-dione
1-(2-Hydroxy-4,6-dimethoxyphenyl)-3-(2,4,5-trimethoxyphenyl)propane-1,3-dione
1-(2-Hydroxy-4,6-dimethoxyphenyl)-3-(3,4,5-trimethoxyphenyl)propane-1,3-dione

### Example 17: synthesis of 2-(3,4-dimethoxyphenyl)-5,7-dimethoxy-4H-chromen-4-one (14)

A suspension of 1-(3,4-dimethoxyphenyl)-3-(2-hydroxy-4,6-dimethoxyphenyl) propane -1,3-dione (7.40 g, 20.53 mmol, 1 eq) in glacial acetic acid (30 mL) was heated at 100°C. To this suspension was added 20 mL of 20% H₂SO₄ in acetic acid and the mixture stirred at 100°C for 10 mins. The mixture was poured into 150 g of crushed ice, which produced a pale-yellow gelatinous solid. The solid was collected, allowed to partially dry by drawing air through the funnel, and then partitioned between 300 mL each of CHCl₃ and distillated water. The organic layer was separated and washed with 150 mL each of 5% NaHCO₃ and saturated brine, dried over MgSO₄, filtered and evaporated to afford a pale yellow solid. Recrystallization from 50 mL of acetone provided 6.05 g (86%) of white needles.
**Molecular Weight:** 342.34 (C₁₉H₁₈O₆).

**¹H-NMR** δ (CDCl₃, 300 MHz) ppm (*J* in Hz): 3.86 (s, 3H, MeO), 3.90 (s, 6H, MeOx2), 3.92 (s, 3H, MeO), 6.30 (d, 1H, *J*=2.1, H-6), 6.48 (d, 1H, *J*=2.1, H-8), 6.53 (s, 1 H, H-3), 6.88 (d, 1 H, *J*=8.6, H-5'), 7.24 (d, 1 H, *J*=2.1, H-2'), 7.42 (dd, 1 H, *J*=2.1, *J*=8.6, H-6') .

**¹³C-NMR** δ (CDCl₃, 75 MHz) ppm: 55.8 (MeO), 56.1 (MeOx2), 56.4 (MeO), 92.9 (C-6), 96.1 (C-8), 107.8 (C-3), 108.5 (C-2'), 109.1 (C-4a), 111.0 (C-5'), 119.5 (C-6'), 123.9 (C-1'), 149.2 (C-4'), 151.7 (C-3'), 159.8 (C-8a), 160.6 (C-5), 160.8 (C-2), 163.9 (C-7), 177.6 (C-4).

Following the method of Example 17, the following compounds shown in Process C are prepared:
5,7-Dimethoxy-2-(4-methoxyphenyl)-4*H*-chromen-4-one
2-(4-Benzyloxy-3-methoxyphenyl)-5,7-dimethoxy-4*H*-chromen-4-one
5,7-Dimethoxy-2-(2,4,5-trimethoxyphenyl)-4*H*-chromen-4-one
5,7-Dimethoxy-2-(3,4,5-trimethoxyphenyl)-4*H*-chromen-4-one

### Example 18: synthesis of 2-(3,4-dimethoxyphenyl)-5-hydroxy-7-methoxy-4H-chromen-4-one (15)

To a solution of 2-(3,4-dimethoxyphenyl)-5,7-dimethoxy-4*H*-chromen-4-one (6.03 g, 17.61 mmol, 1eq) in dry dichloromethane (75 mL) at 0°C was added dropwise a solution of boron tribromide 1M in dichloromethane (17.61 mL, 17.61 mmol, 1eq). After vigorous stirring at room temperature overnight, the resulting mixture was quenched with ethanol (150 mL) then the solvent was concentrated in vacuo. The yellow solid obtained, was refluxed in ethanol/water (1/1) (300 mL). The solution placed at 0°C, filtered, washed with diethyl ether (100 mL) to afford 4.75 g (82%) of a yellow solid.
**Molecular Weight:** 328.32 (C₁₈H₁₆O₆).

**¹H-NMR** δ (CDCl₃, 300 MHz) ppm (*J* in Hz): 3.86 (s, 3H, MeO), 3.95 (s, 3H, MeO), 3.96 (s, 3H, MeO), 6.33 (d, 1H, *J*=2.3, H-6), 6.45 (d, 1H, *J*=2.3, H-8), 6.54 (s, 1H, H-3), 6.94 (d, 1H, *J*=8.6, H-5'), 7.29 (d, 1H, *J*=2.1, H-2'), 7.48 (dd, 1H, *J*=2.1, *J*=8.6, H-6') .

**¹³C-NMR** δ (CDCl₃, 75 MHz) ppm: 56.2 (MeOx2), 55.9 (MeO), 92.7 (C-8), 98.2 (C-6), 104.7 (C-3), 105.6 (C-4a), 108.8 (C-2'), 111.2 (C-5'), 120.2 (C-6'), 123.8 (C-1'), 149.4 (C-4'), 152.4 (C-3'), 157.7 (C-8a), 162.2 (C-5), 164.0 (C-2), 165.5 (C-7), 182.4 (C-4).

Following the method of Example 18, the following compounds shown in Process C are prepared:
5-Hydroxy-7-methoxy-2-(4-methoxyphenyl)-4*H*-chromen-4-one
2-(4-Benzyloxy-3-methoxyphenyl)-5-hydroxy-7-methoxy-4*H*-chromen-4-one
5-Hydroxy-7-methoxy-2-(2,4,5-trimethoxyphenyl)-4*H*-chromen-4-one
5-Hydroxy-7-methoxy-2-(3,4,5-trimethoxyphenyl)-4*H*-chromen-4-one

### Example 19: synthesis of 2-(3,4-dimethoxyphenyl)-5-hydroxy-6-iodo-7-methoxy-4H-chromen-4-one (16)

To a solution of 2-(3,4-dimethoxyphenyl)-5-hydroxy-7-methoxy-4*H*-chromen-4-one (4.40 g, 13.40 mmol, 1 eq) in dichloromethane-MeOH (5/2) (210 mL), calcium carbonate (9.39 g, 93.81 mmol, 7 eq) and benzyltrimethyl ammonium chloroiodate (BTMA-ICl₂) (4.66 g, 13.40 mmol, 1 eq) were added under argon at 0°C. After vigorous stirring at room temperature overnight, the resulting mixture was quenched with water (150 mL) and extracted three times with dichloromethane (200 mL). The combined organic phases were washed with brine (150 mL), dried over MgSO₄ and concentrated in vacuo. The solid obtained was recristallized from ethanol (150 mL) to afford 5.29 g (87%) of a yellow solid.
**Molecular Weight:** 454.21 (C₁₈H₁₅IO₆).

**¹H-NMR** δ (CDCl₃, 300 MHz) ppm (*J* in Hz): 3.96 (s, 3H, MeO), 3.98 (s, 3H, MeO), 3.99 (s, 3H, MeO), 6.51 (s, 1H, H-8), 6.61 (s, 1H, H-3), 6.96 (d, 1H, *J*=8.6, H-5'), 7.31 (d, 1H, *J*=2.1, H-2'), 7.50 (dd, 1H, *J*=2.1, *J*=8.6, H-6'), 13.79 (s, 1H, OH).

**¹³C-NMR** δ (CDCl₃, 75 MHz) ppm: 56.3 (MeOx2), 57.1 (MeO), 70.1 (C-6), 90.5 (C-8), 104.7 (C-3), 105.7 (C-4a), 108.9 (C-2'), 111.3 (C-5'), 120.4 (C-6'), 123.5 (C-1'), 149.5 (C-4'), 152.6 (C-3'), 158.2 (C-8a), 161.2 (C-5), 163.4 (C-2), 164.3 (C-7), 181.5 (C-4).

Following the method of Example 19, the following compounds shown in Process C are prepared:
5-Hydroxy-6-iodo-7-methoxy-2-(4-methoxyphenyl)-4*H*-chromen-4-one
2-(4-Benzyloxy-3-methoxyphenyl)-5-hydroxy-6-iodo-7-methoxy-4*H*-chromen-4-one
5-Hydroxy-6-iodo-7-methoxy-2-(2,4,5-trimethoxyphenyl)-4*H*-chromen-4-one
5-Hydroxy-6-iodo-7-methoxy-2-(3,4,5-trimethoxyphenyl)-4*H*-chromen-4-one

### Example 20: synthesis of 2-(3,4-dimethoxyphenyl)-6-iodo-5,7-dimethoxy-4H-chromen-4-one (17)

To a solution of 2-(3,4-dimethoxyphenyl)-5-hydroxy-6-iodo-7-methoxy-4*H-*chromen-4-one (0.63 g, 1.38 mmol, 1 eq) in THF (30 mL), potassium carbonate (0.38 g, 2.77 mmol, 2 eq) and dimethylsulfate (0.13 mL, 1.39 mmol, 1 eq) were added under argon at 0°C. After vigorous stirring at reflux for 3h, the resulting mixture was filtered and the filtrate concentrated in vacuo. The yellow solid obtained was purified by silica gel column chromatography (ethyl acetate /heptanes: 1 /4, 3/7 then 1/1) to afford 0.43 g (66%) of a white solid.
**Molecular Weight:** 468.24 (C₁₉H₁₇IO₆).

**¹H-NMR** δ (CDCl₃, 300 MHz) ppm (*J* in Hz): 3.94 (s, 3H, MeO), 3.96 (s, 3H, MeO), 3.98 (s, 3H, MeO), 4.01 (s, 3H, MeO), 6.63 (s, 1H, H-3), 6.77 (s, 1H, H-8), 6.96 (d, 1 H, *J*=8.5, H-5'), 7.30 (d, 1H, *J*=2.1, H-2'), 7.50 (dd, 1H, *J*=2.1, *J*=8.5, H-6') .

**¹³C-NMR** δ (CDCl₃, 75 MHz) ppm: 56.2 (MeO), 56.3 (MeO), 57.1 (MeO), 62.0 (MeO), 83.2 (C-6), 96.1 (C-8), 107.7 (C-3), 108.8 (C-2'), 111.3 (C-5'), 119.9 (C-6'), 123.8 (C-1'), 149.4 (C-4'), 152.1 (C-3'), 159.8 (C-8a), 160.4 (C-5), 161.4 (C-2), 162.1 (C-7), 176.0 (C-4).

Following the method of Example 20, the following compounds shown in Process C are prepared:
6-Iodo-5,7-dimethoxy-2-(4-methoxyphenyl)-4*H*-chromen-4-one
2-(4-Benzyloxy-3-methoxyphenyl)-6-iodo-5,7-dimethoxy-4*H*-chromen-4-one
6-Iodo-5,7-dimethoxy-2-(2,4,5-trimethoxyphenyl)-4*H*-chromen-4-one
6-Iodo-5,7-dimethoxy-2-(3,4,5-trimethoxyphenyl)-4*H*-chromen-4-one

### Example 21: synthesis of 6-(12-benzyloxydodec-1-ynyl)-2-(3,4-dimethoxyphenyl)-5,7-dimethoxy-4H-chromen-4-one (18)

To a solution of 2-(3,4-dimethoxyphenyl)-6-iodo-5,7-dimethoxy-4*H*-chromen-4-one (0.25 g, 0.53 mmol, 1 eq) in DMF (5 mL), piperidine (0.11 mL, 1.07 mmol, 2 eq), (Ph₃P)₂PdCl₂ (0.03 g, 0.04 mmol, 0.07 eq), Cul (0.007 g, 0.04 mmol, 0.07 eq) then dodec-11-ynyloxymethylbenzene (0.22 g, 0.80 mmol, 1.5 eq) were added under argon at room temperature. The reaction was stirred at 80°C overnight. The resulting mixture was quenched with chilled water (50 mL) and extracted three times with ethyl acetate (50 mL). The combined organic layers were washed with brine, dried over MgSO₄ and concentrated in vacuo. The residue was purified by silica gel column chromatography (ethyl acetate / heptanes: 1 /4, 3/7 then 1/1) to afford 0.22 g (66%) of a fatty colourless oil.
**Molecular Weight:** 612.77 (C₃₈H₄₄O₇).

**¹H-NMR** δ (CDCl₃, 300 MHz) ppm (*J* in Hz): 1.30 (s large, 12H, H-5" to H-10"), 1.42 to 1.70 (m, 4H, H-4",11"), 2.53 (t, 2H, *J*=6.9, H-3"), 3.46 (t, 2H, *J*=6.6, H-12"), 3.96 (s, 3H, MeO), 3.97 (s, 3H, MeO), 3.98 (s, 3H, MeO), 4.02 (s, 3H, MeO), 4.50 (s, 2H, C*H*₂Ph), 6.58 (s, 1H, H-8), 6.72 (s, 1H, H-3), 6.96 (d, 1H, *J*=8.7, H-5'), 7.33 (m, 6H, H-2', C*H*₂*Ph*), 7.49 (dd, 1H, *J*=2.4, *J*=8.7, H-6').

**¹³C-NMR** δ (CDCl₃, 75 MHz) ppm: 20.1 (C-3"), 26.3 (C-10"), 28.8 to 29. 9 (C-4" to C-9", C-1 1 "), 56.2 (MeO), 56.2 (MeO), 56.6 (MeO), 61.9 (MeO), 70.6 (C-12"), 71.2 (C-1 "), 73.0 (CH₂Ph), 95.5 (C-8), 100.1 (C-2"), 106.9 (C-4a), 107.9 (C-3), 108.7 (C-2'), 111.2 (C-5'), 112.8 (C-6), 119.7 (C-6'), 124.0 (C-1'), 127.5 (CH₂*Ph*), 127.7 (CH₂*Ph*), 128.4 (CH₂*Ph*), 132.3 (CH₂*Ph*), 149.3 (C-4'), 151.9 (C-3'), 158.1 (C-8a), 161.1 (C-5), 162.5 (C-2), 163.7 (C-7), 176.5 (C-4).

Following the method of Example 21, the following compounds shown in Process C are prepared:
6-(12-Benzyloxydodec-1-ynyl)-5,7-dimethoxy-2-(4-methoxyphenyl)-4*H-*chromen-4-one
6-(13-Benzyloxytridec-1-ynyl)-5,7-dimethoxy-2-(4-methoxyphenyl)-4*H*-chromen-4-one
6-(13-Benzyloxytridec-1-ynyl)-2-(3,4-dimethoxy-phenyl)-5,7-dimethoxy-4*H-*chromen-4-one
2-(4-Benzyloxy-3-methoxyphenyl)-6-(12-benzyloxydodec-1-ynyl)-5,7-dimethoxy-4*H*-chromen-4-one
2-(4-Benzyloxy-3-methoxyphenyl)-6-(13-benzyloxytridec-1-ynyl)-5,7-dimethoxy-4*H*-chromen-4-one
6-(12-Benzyloxydodec-1-ynyl)-5,7-dimethoxy-2-(2,4,5-trimethoxyphenyl)-4*H-*chromen-4-one
6-(13-Benzyloxytridec-1-ynyl)-5,7-dimethoxy-2-(2,4,5-trimethoxyphenyl)-4*H-*chromen-4-one
6-(12-Benzyloxydodec-1-ynyl)-5,7-dimethoxy-2-(3,4,5-trimethoxyphenyl)-4*H-*chromen-4-one
6-(13-Benzyloxytridec-1-ynyl)-5,7-dimethoxy-2-(3,4,5-trimethoxyphenyl)-4*H-*chromen-4-one

### Example 22: synthesis of 6-(12-hydroxydodec-1-yl)-2-(3,4-dimethoxyphenyl)-5,7-dimethoxy-4H-chromen-4-one (19)

To a solution of 6-(12-benzyloxy-dodec-1-ynyl)-2-(3,4-dimethoxy-phenyl)-5,7-dimethoxy-4*H*-chromen-4-one (0.22 g, 0.35 mmol, 1 eq) in EtOH / acetone /water (10/10/1) (10.5 mL), was added 10% Pd/C (0.3 g, 136% ^{w}/_{w}). The reaction mixture was then stirred under hydrogen atmosphere for 36 h at room temperature. The solution was filtered through Celite and concentrated *in vacuo*. The residue was purified by silica gel column chromatography (ethyl acetate / heptanes: 1/1 then 4/1) to afford 0.10 g (53%) of a white solid.
**Molecular Weight:** 526.66 (C₃₁H₄₂O₇).

**¹H-NMR** δ (CDCl₃, 300 MHz) ppm (*J* in Hz): 1.25 (s large, 16H, H-3" to H-10"), 1.52 to 1.60 (m, 4H, H-2",11"), 2.66 (t, 2H, *J*=7.8, H-1"), 3.63 (t, 2H, *J*=6.6, H-12"), 3.89 (s, 3H, MeO), 3.93 (s, 3H, MeO), 3.95 (s, 3H, MeO), 3.98 (s, 3H, MeO), 6.60 (s, 1H, H-8), 6.74 (s, 1H, H-3), 6.97 (d, 1H, *J*=8.7, H-5'), 7.33 (d, 1H, *J*=2.4, H-2'), 7.50 (dd, 1H, *J*=2.4, *J*=8.7, H-6').

**¹³C-NMR** δ (CDCl₃, 75 MHz) ppm: 23.3 (C-1"), 25.9 (C-10"), 29.6 to 30.0 (C-2" to C-9"), 33.0 (C-11 "), 56.1 (MeO), 56.2 (MeO), 56.3 (MeO), 62.6 (MeO), 63.2 (C-12"), 95.3 (C-8), 107.9 (C-3), 108.8 (C-2'), 111.2 (C-5'), 112.3 (C-4a), 119.7 (C-6'), 123.5 (C-6), 124.4 (C-1'), 149.4 (C-4'), 151.8 (C-3'), 157.7 (C-8a), 157.8 (C-5), 161.0 (C-2), 162.4 (C-7), 177.2 (C-4).

Following the method of Example 22, the following compounds shown in Process C are prepared:
6-(12-Hydroxydodecyl)-5,7-dimethoxy-2-(4-methoxyphenyl)-4*H*-chromen-4-one
6-(13-Hydroxytridecyl)-5,7-dimethoxy-2-(4-methoxyphenyl)-4*H*-chromen-4-one
6-(13-Hydroxytridec-1-yl)-2-(3,4-dimethoxyphenyl)-5,7-dimethoxy-4*H*-chromen-4-one
2-(4-Hydroxy-3-methoxyphenyl)-6-(12-hydroxydodecyl)-5,7-dimethoxy-4*H-*chromen-4-one
2-(4-Hydroxy-3-methoxyphenyl)-6-(13-hydroxytridecyl)-5,7-dimethoxy-4*H-*chromen-4-one
6-(12-Hydroxydodecyl)-5,7-dimethoxy-2-(2,4,5-trimethoxyphenyl)-4*H*-chromen-4-one
6-(13-Hydroxytridecyl)-5,7-dimethoxy-2-(2,4,5-trimethoxyphenyl)-4*H*-chromen-4-one
6-(12-Hydroxydodecyl)-5,7-dimethoxy-2-(3,4,5-trimethoxyphenyl)-4*H*-chromen-4-one
6-(13-Hydroxytridecyl)-5,7-dimethoxy-2-(3,4,5-trimethoxyphenyl)-4*H*-chromen-4-one

### Example 23: synthesis of 2-(3,4-dihydroxyphenyl)-5-hydroxy-6-(12-hydroxydodecyl)-7-methoxy-4H-chromen-4-one (20)

To a solution of 6-(12-hydroxydodec-1-yl)-2-(3,4-dimethoxyphenyl)-5,7-dimethoxy-4*H*-chromen-4-one (0.02 g, 0.04 mmol, 1 eq) in dichloromethane (5 mL) at 0°C, was added boron tribromide in dichloromethane (1.0 M, 0.5 mL, 0.49 mmol, 12 eq). The mixture was warmed to room temperature and then stirred for 22 h. The reaction mixture was then cooled to 0°C and methanol (5 mL) was added. The reaction mixture was heated to reflux for 2 h, and then concentrated *in vacuo* to give a yellow solid. Water (30 mL) was added and the aqueous phase was extracted with ethyl acetate (3*30 mL). The combined organic layers were washed with brine, dried over magnesium sulfate and concentrated *in vacuo*. The residue was precipitated from aqueous ethanol to give 0.01 g (50%) of a pale-yellow solid.
**Molecular Weight:** 484.59 (C₂₈H₃₆O₇).

**¹H-NMR** δ (acetone-d6, 300 MHz) ppm (*J* in Hz): 1.30 (s large, 16H, H-3" to H-10"), 1.56 to 1.83 (m, 4H, H-2",11"), 2.64 (t, 2H, *J*=7.2, H-1"), 3.48 (t, 2H, *J*=6.9, H-12"), 3.99 (s, 3H, MeO), 6.62 (s, 1H, H-3), 6.77 (s, 1H, H-8), 7.01 (d, 1H, *J*=8.7, H-5'), 7.50 (m, 2H, H-2', 6').

Following the method of Example 23, the following compounds shown in Process C are prepared:
5-Hydroxy-6-(12-hydroxydodecyl)-2-(4-hydroxyphenyl)-7-methoxy-4*H-*chromen-4-one
5-Hydroxy-2-(4-hydroxyphenyl)-6-(13-hydroxytridecyl)-7-methoxy-4*H*-chromen-4-one
2-(3,4-Dihydroxyphenyl)-5-hydroxy-6-(13-hydroxytridecyl)-7-methoxy-4*H-*chromen-4-one
5-Hydroxy-6-(12-hydroxydodecyl)-7-methoxy-2-(2,4,5-trihydroxyphenyl)-4*H-*chromen-4-one
5-Hydroxy-6-(13-hydroxytridecyl)-7-methoxy-2-(2,4,5-trihydroxyphenyl)-4*H-*chromen-4-one
5-Hydroxy-6-(12-hydroxydodecyl)-7-methoxy-2-(3,4,5-trihydroxyphenyl)-4*H-*chromen-4-one
5-Hydroxy-6-(13-hydroxytridecyl)-7-methoxy-2-(3,4,5-trihydroxyphenyl)-4*H-*chromen-4-one
5,7-Dihydroxy-6-(12-hydroxydodecyl)-2-(4-hydroxyphenyl)-4*H*-chromen-4-one
5,7-Dihydroxy-2-(4-hydroxyphenyl)-6-(13-hydroxytridecyl)-4*H*-chromen-4-one
2-(3,4-Dihydroxyphenyl)-5,7-dihydroxy-6-(12-hydroxydodecyl)-4*H*-chromen-4-one
2-(3,4-Dihydroxyphenyl)-5,7-dihydroxy-6-(13-hydroxytridecyl)-4*H*-chromen-4-one
5,7-Dihydroxy-6-(12-hydroxydodecyl)-2-(2,4,5-trihydroxyphenyl)-4*H*-chromen-4-one
5,7-Dihydroxy-6-(13-hydroxytridecyl)-2-(2,4,5-trihydroxyphenyl)-4*H*-chromen-4-one
5,7-Dihydroxy-6-(12-hydroxydodecyl)-2-(3,4,5-trihydroxyphenyl)-4*H*-chromen-4-one
5,7-Dihydroxy-6-(13-hydroxytridecyl)-2-(3,4,5-trihydroxyphenyl)-4*H*-chromen-4-one

### Example synthesis of compound of general Formula (II)

### Example 24: synthesis of 2-(10-benzyloxydecyl)-7-(3,4-dimethoxyphenyl)-4,8-dimethoxy-9H-furo[2,3-f]chromen-9-one (21)

To a solution of 2-(3,4-dimethoxyphenyl)-6-iodo-3,5,7-trimethoxy-4*H*-chromen-4-one (0.10 g, 0.20 mmol, 1 eq) in piperidine (5 mL), PdCl₂P(Ph₃)₂ (0.01 g, 0.014 mmol, 0.07 eq) and Cul (0.003 g, 0.014 mmol, 0.07 eq) were added under argon, followed by dodec-11-ynyloxymethylbenzene (0.08 g, 0.30 mmol, 1.5 eq). The reaction was stirred at 80°C overnight. The resulting mixture was quenched with crushed ice, extracted three times with ethyl acetate (50 mL). The combined organic phases were washed with brine, dried over MgSO₄ and concentrated *in vacuo*. The residue was purified by silica gel column chromatography (ethyl acetate / heptanes: 1 /4, 3/7 then 1/1) to afford 0.08 g (62%) of a pale yellow solid.
**Molecular Weight:** 628.75 (C₃₈H₄₄O₈).

**¹H-NMR** δ (CDCl₃, 300 MHz) ppm (J in Hz): 1.30 (s large, 8H, H-5" to H-8"), 1.41 to 1.70 (m, 4H, H-4",9"), 2.90 (t, 2H, *J*=7.5, H-3"), 3.46 (t, *J*=6.6, H-10")), 3.87 (s, 3H, MeO), 3.97 (s, 9H, MeOx3), 4.04 (s, 3H, MeO), 4.50 (s, 2H, C*H*₂Ph), 6.68 (s, 1H, H-5), 6.74 (s, 1H, H-2), 6.98 (d, 1H, *J*=9.0, H-5'), 7.33 (m, 5H, CH₂*Ph*), 7.70 (d, 1H, *J*=2.1, H-2'), 7.71 (dd, 1H, *J*=2.1, *J*=9.0, H-6').

Following the method of Example 24, the following compounds shown in Process D are prepared:
2-(11-Benzyloxyundecyl)-7-(3,4-dimethoxyphenyl)-4,8-dimethoxy-9*H*-furo[2,3-f]chromen-9-one
2-(12-Benzyloxydodecyl)-7-(3,4-dimethoxyphenyl)-4,8-dimethoxy-9*H*-furo[2,3-f]chromen-9-one
2-(13-Benzyloxytridecyl)-7-(3,4-dimethoxyphenyl)-4,8-dimethoxy-9*H*-furo[2,3-f]chromen-9-one
2-(14-Benzyloxytetradecyl)-7-(3,4-dimethoxyphenyl)-4,8-dimethoxy-9*H-*furo[2,3-f]chromen-9-one

### Example 25: synthesis of 2-(10-benzyloxydecyl)-7-(3,4-dimethoxyphenyl)-4-methoxy-9H-furo[2,3-f]chromen-9-one (25)

To a solution of 2-(3,4-dimethoxyphenyl)-6-iodo-5,7-dimethoxy-4*H*-chromen-4-one (0.41 g, 0.89 mmol, 1 eq) in piperidine (5 mL), PdCl₂P(Ph₃)₂ (0.044 g, 0.062 mmol, 0.07 eq) and Cul (0.012 g, 0.062 mmol, 0.07 eq) were added followed by dodec-11-ynyloxymethylbenzene (0.36 g, 1.33 mmol, 1.5 eq). The reaction was stirred at 100°C overnight. The resulting mixture was quenched with chilled ice water, extracted three times with ethyl acetate (50 mL). The combined organic phases were washed with brine, dried over MgSO₄. and concentrated *in vacuo*. The residue was purified by silica gel column chromatography (ethyl acetate / heptanes: 1 /4, 3/7 then 1/1) to afford 0.55 g (44%) of a pale yellow solid.
**Molecular Weight:** 598.73 (C₃₇H₄₂O₇).

**¹H-NMR** δ (CDCl₃, 300 MHz) ppm (*J* in Hz): 1.30 (s large, 8H, H-5" to H-8"), 1.41 to 1.70 (m, 4H, H-4",9"), 2.90 (t, 2H, *J*=7.5, H-3"), 3.46 (t, *J*=6.6, H-10"), 3.94 (s, 3H, MeO), 3.97 (s, 3H, MeO), 4.01 (s, 3H, MeO), 4.48 (s, 2H, C*H*₂Ph), 6.49 (s, 1H, H-5), 6.77 (s, 1H, H-8), 6.79 (s, 1H, H-3), 6.93 (d, 1H, *J*=9.0, H-5'), 7.33 (m, 5H, CH₂*Ph*), 7.70 (d, 1H, *J*=2.1, H-2'), 7.71 (dd, 1H, *J*=2.1, *J*=9.0, H-6').

Following the method of Example 25, the following compounds shown in Process E are prepared:
2-(11-Benzyloxyundecyl)-7-(3,4-dimethoxyphenyl)-4-methoxy-9*H*-furo[2,3-f]chromen-9-one
2-(12-Benzyloxydodecyl)-7-(3,4-dimethoxyphenyl)-4-methoxy-9*H*-furo[2,3-f]chromen-9-one
2-(13-Benzyloxytridecyl)-7-(3,4-dimethoxyphenyl)-4-methoxy-9*H*-furo[2,3-f]chromen-9-one
2-(14-Benzyloxytetradecyl)-7-(3,4-dimethoxyphenyl)-4-methoxy-9*H*-furo[2,3-f]chromen-9-one
7-(4-Benzyloxy-3-methoxyphenyl)-2-(10-benzyloxydecyl)-4-methoxy-9*H-*furo[2,3-f]chromen-9-one
7-(4-Benzyloxy-3-methoxyphenyl)-2-(11-benzyloxyundecyl)-4-methoxy-9*H-*furo[2,3-f]chromen-9-one
7-(4-Benzyloxy-3-methoxyphenyl)-2-(12-benzyloxydodecyl)-4-methoxy-9*H-*furo[2,3-f]chromen-9-one
7-(4-Benzyloxy-3-methoxyphenyl)-2-(13-benzyloxytridecyl)-4-methoxy-9*H-*furo[2,3-f]chromen-9-one
7-(4-Benzyloxy-3-methoxyphenyl)-2-(14-benzyloxytetradecyl)-4-methoxy-9*H-*furo[2,3-f]chromen-9-one

### Example 26: synthesis of 7-(3,4-dimethoxyphenyl)-2-(10-hydroxydecyl)-4-methoxy-9H-furo[2,3-f]chromen-9-one (26)

To a solution of 2-(10-benzyloxydecyl)-7-(3,4-dimethoxyphenyl)-4-methoxy-9*H-*furo[2,3-f]chromen-9-one (0.23 g, 0.38 mmol, 1 eq) in EtOH-cyclohexene (4/1) (16 mL) was added 15-20% Pd(OH)₂ / C (0.07 g, 30% ^{w}/_{w}). After vigorous stirring at reflux overnight, the solution was filtered through Celite and concentrated *in vacuo*. The residue was purified by silica gel column chromatography (ethyl acetate / heptanes: 1 /4, 3/7 then 1/1) to afford 0.10 g (52%) of a white solid.
**Molecular Weight:** 508.60 (C₃₀H₃₆O₇).

**¹H-NMR** δ (CDCl₃, 300 MHz) ppm (*J* in Hz): 1.30 (s large, 12H, H-3" to H-8"), 1.52 to 1.70 (m, 4H, H-2",9"), 2.89 (t, 2H, *J*=7.5, H-1 "), 3.64 (t, *J*=6.6, H-10"), 3.96 (s, 3H, MeO), 3.99 (s, 3H, MeO), 4.04 (s, 3H, MeO), 6.52 (s, 1H, H-5), 6.77 (s, 1H, H-8), 6.79 (s, 1H, H-3), 6.98 (d, 1H, *J*=9.0, H-5'), 7.37 (d, 1H, *J*=2.1, H-2'), 7.55 (dd, 1H, *J*=2.1, *J*=9.0, H-6').

Following the method of Example 26, the following compounds shown in Process E are prepared:
7-(3,4-Dimethoxyphenyl)-2-(11-hydroxyundecyl)-4-methoxy-9*H*-furo[2,3-f]chromen-9-one
7-(3,4-Dimethoxyphenyl)-2-(12-hydroxydodecyl)-4-methoxy-9*H*-furo[2,3-f]chromen-9-one
7-(3,4-Dimethoxyphenyl)-2-(13-hydroxytridecyl)-4-methoxy-9*H*-furo[2,3-f]chromen-9-one
7-(3,4-Dimethoxyphenyl)-2-(14-hydroxytetradecyl)-4-methoxy-9*H*-furo[2,3-f]chromen-9-one
7-(4-Hydroxy-3-methoxyphenyl)-2-(10-hydroxydecyl)-4-methoxy-9*H*-furo[2,3-f]chromen-9-one
7-(4-Hydroxy-3-methoxyphenyl)-2-(11-hydroxyundecyl)-4-methoxy-9*H*-furo[2,3-f]chromen-9-one
7-(4-Hydroxy-3-methoxyphenyl)-2-(12-hydroxydodecyl)-4-methoxy-9*H*-furo[2,3-f]chromen-9-one
7-(4-Hydroxy-3-methoxyphenyl)-2-(13-hydroxytridecyl)-4-methoxy-9*H*-furo[2,3-f]chromen-9-one
7-(4-Hydroxy-3-methoxyphenyl)-2-(14-hydroxytetradecyl)-4-methoxy-9*H-*furo[2,3-f]chromen-9-one

### Biological Assays

### Inhibition of the activation of microglial cells

The experiments that were carried out concern the ability of these molecules to inhibit the liberation of nitrites and Tumor Necrosis Factor-alpha (TNF-α) in the activated microglia. NO and TNF-α production are two common parameters related to inflammatory processes.

### Example 27: nitrite quantification by Griess method

To evaluate the nitrite production, microglial cells were cultured in DMEM-F12 medium containing 2% heat-inactivated FBS in 48 well plates at a density of 20000 cells per well. Peripheral wells of the plate were discarded. After 72h, 400µL out of 500µL medium was replaced by 350µL fresh medium. The cells were treated with each molecule to be tested by adding 50µL of a 10X solution to obtain a final concentration ranging from 0.1µM to 10µM. Each treatment was done in duplicates. 10ng/ml LPS was added after 1 hour and the medium was collected after 48h and stored at -20°C until analysis. To quantify the NO production, nitrites were measured using Griess reactive (sulfanilamide 1%, Sigma ; N-(1-Naphthyl) ethylenediamine 0,1%, Sigma ; phosphoric acid 2,5%). Briefly, 100µL of medium was placed into a 96 well plate and 100µL of Griess reactive was added. After 10mn incubation at RT in the dark, the OD was measured at 550nm.

The ability of each test compound to decrease the production of nitrite is presented as a percent decrease in OD with respect to the absorbance of the control condition i.e. 0.1% DMSO. Examples of the results are shown in Table 1.

| **Test compound at 1µM** | **% of NO/control** |
|---|---|
| 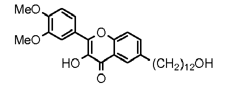 | 87.7 ± 13.3 |
| 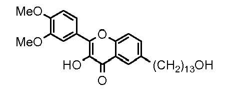 | 89.0 ± 13.1 |
| 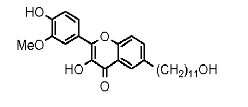 | 72.0 ± 6.1 |
| 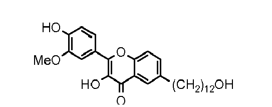 | 59.8 ± 7.4 |
| 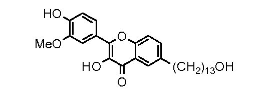 | 53.7 ± 7.3 |
| 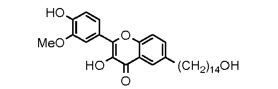 | 77.5 ± 7.9 |
| 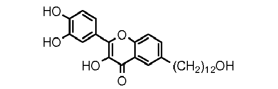 | 74.7 ± 9.9 |
| 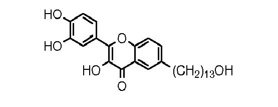 | 92.8 ± 3.5 |

### Example 28: TNF-α quantification by ELISA

To quantify the presence of TNF-α in the supernatant of microglial cells, the medium was collected 48h after treatment as described above. The ELISA 96 well plate was incubated overnight with the capture antibody (0.8µg/ml, R&D). 10µl of each sample was diluted with 90µL of incubation buffer (PBS - BSA 1%, pH 7.2-7.4) and the plate was incubated for 2h at RT. The plate was washed 3 times and incubated for 2h with the detection antibody (150ng/mL, R&D). After another washing step, the plate was incubated with streptavidine-HRP (20mn) and the peroxydase revealed with the R&D detection kit. After 20mn incubation at RT, the reaction was stopped with an equal volume of a 10% solution of sulfuric acid and the OD was measured at 450nm.

The ability of each test compound to decrease the production of TNF-α is presented as a percent decrease in OD with respect to the absorbance of the control condition i.e. 0.1% DMSO. Examples of the results are shown in Table 2.

| **Test compound at 1µM** | **% of TNF-α/control** |
|---|---|
| 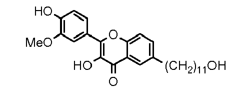 | 69.2 ± 3.9 |
| 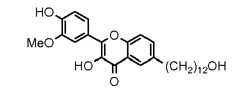 | 64.7 ± 2.8 |
| 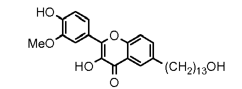 | 77.2 ± 9.4 |
| 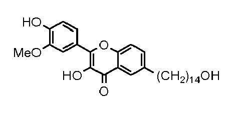 | 75.4 ± 17.2 |

### Example 29: RNA extraction, reverse transcription and real-time PCR

To evaluate mRNA expression levels, total RNA was extracted using invisorb RNA extraction kit (Invitek). The concentration was determined by reading the OD at 260nm. Retro-transcription was performed using Im-Prom-II™ Reverse Transcription System (Promega) with 1µg of total RNA.

The primer pairs for mouse iNOS, TNF-α and beta-actin were designed with the beacon designer software (premierbiosoft).

| **Gene (Accession No.)** | Forward Primer | Reverse Primer |
|---|---|---|
| **Beta-actin** *(NM*_*007393)* | 5'- AGGGAAATCGTGCGTGAC ATCAAAGAG-3' | 5'- GGAGGAAGAGGATGCGGCA GTGG-3' |
| **TNF-**α *(NM*_*013693)* | 5'- GGTTCTGTCCCTTTCACTCA C-3' | 5'-TGCCTCTTCTGCCAGTTCC-3' |
| **iNOS** *(NM*_*010927)* | 5' AGCCCTCACCTACTTCCT G-3' | 5'- CAATCTCTGCCTATCCGTCTC-3' |

The reaction mixture contained 12.5µl 2X SYBR Green mastermix (Biorad), 1µl cDNA, 1µl of each primer (12.5µM) and 9.5µl of H₂0. The real-time PCR amplification was controlled by the MyIQ5 system (Biorad). After activation of the enzyme at 94°C for 3mn, the amplification was done by cycling 40 times between a denaturation step at 94°C and the annealing step at 54.5°C. Gene expression was analyzed using the provided software and normalized to beta-actin expression.

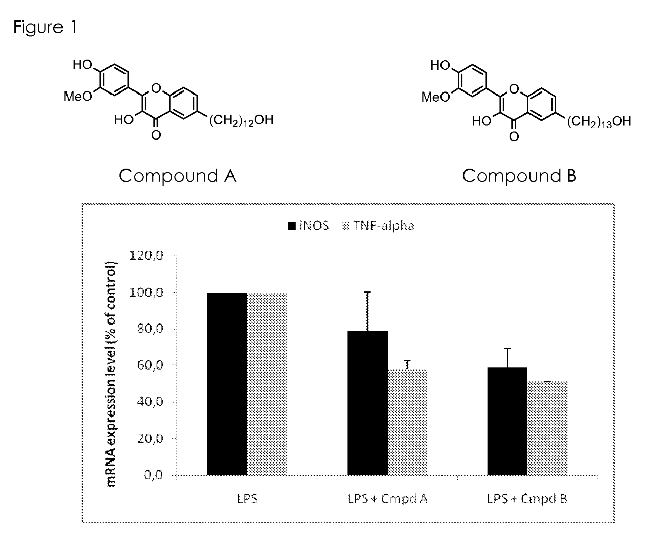

The results showed that compounds according to the invention are able to significantly down-regulate the iNOS and TNF-α gene expression in microglial cells as shown in figure 1.

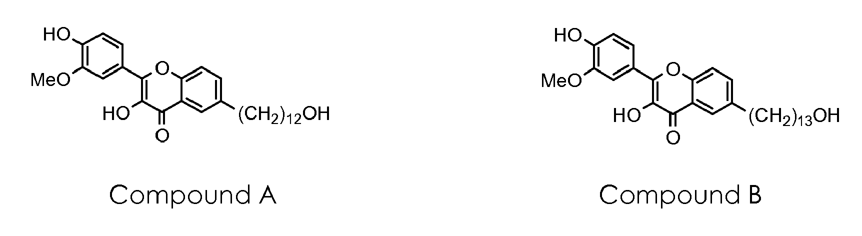

Fig. 1: Compound A and B inhibits microglial activation in vitro. Real-time PCR gene expression analysis in microglial cell line. RNA extraction was performed after 6 hours of incubation with compound A and B (1 µM), with or without LPS activation (0.01 µg/ml). Expression of iNOS and TNF-α transcripts in treated microglial cells are significantly decreased. β-actin was used as an internal control.

### Cell differentiation

The experiments that were carried out concern the ability of these molecules to induce the differentiation of NSCs towards the neuronal lineage and the differentiation of human neuroblastoma cell line into neurons by inhibiting the Notch signaling pathway. Moreover, if these compounds modulate cell differentiation through inhibition of the Notch signaling pathway, they can be useful for the treatment of cancer where the Notch signaling is up-regulated.

### Example 30: Differentiation of NSCs to neurons

The NSCs derived neurospheres culture was established from embryonic mouse cortices. Neurospheres were maintained in Neurobasal-A medium supplemented with B27 w/o retinoic acid, 2mM Glutamine, 20ng/ml EGF and antibiotics. The neurospheres were dissociated using a non enzymatic dissociation medium (Invitrogen) and the single cell suspension added to a flask with fresh medium. To differentiate the neurospheres into neurons, oligodendrocytes and astrocytes, 3 to 4 days old neurospheres were cultured in poly-ornithine treated 35mm dishes. Cells were treated with test compounds (1 µM) or 0.1% DMSO vehicle as control in Neurobasal-A medium supplemented with B27 w/o retinoic acid, 2mM Glutamine, antibiotics, 2ng/ml EGF and 0.5% of heat inactivated FBS. RNA extraction was performed after 24 hours of incubation and gene expression was analyzed by real-time PCR.

The primer pairs for mouse Mash1, Hes5 and beta-actin were designed with the beacon designer software (premierbiosoft).

| **Gene (Accession No.)** | Forward Primer | Reverse Primer |
|---|---|---|
| **Beta-actin** *(NM*_*007393)* | 5'- AGGGAAATCGTGCGTGACATC AAAGAG-3' | 5'- GGAGGAAGAGGATGCGGCA GTGG-3' |
| **Mash1** *(NM*_*008553)* | 5'-AAGATGAGCAAGGTGGAG- 3' | 5'-AGTCGTTGGAGTAGTTGG-3' |
| **Hes5** *(NM*_*0*10419) | 5'-CGGTGGTGGAGAAGATGC- 3' | 5'-CTTGGAGTTGGGCTGGTG-3' |

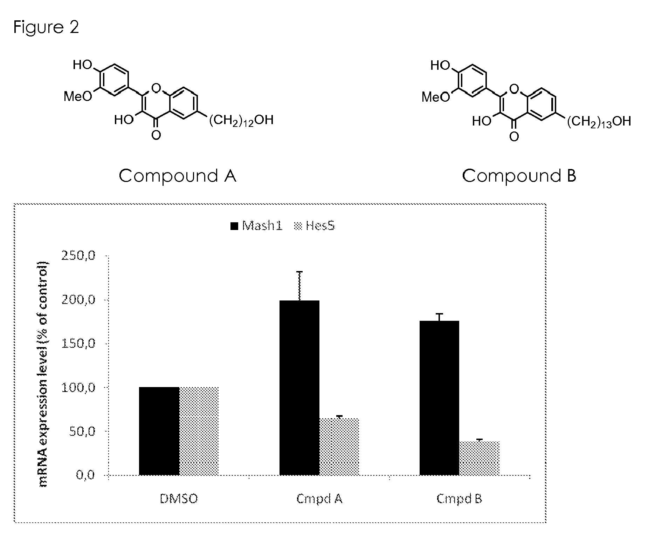

Notch signaling controls a wide variety of cell fate decisions, including the differentiation of NSCs during neurogenesis [Artavanis-Tsakonas, S. et al., Science, 1999, 284, 770]. Hence, compounds inhibiting the Notch signaling pathway would increase the production of neurons from NSCs. Notch activation induces the expression of the downstream effectors Hes1 and Hes5, which down-regulate gene transcription of b-HLH transcription factors such as Mash1. Therefore, we tested whether compounds of general Formula (I) and (II) could modulate Notch signalling in NSCs. Interestingly, Hes5 expression, assessed by real-time PCR, were decreased in NSCs cultures treated with example compounds A and B at 1 µM (figure 2). Inhibition of the Hes5 gene expression in the treated cultures was further correlated with an increase of Mashl1 transcripts, a b-HLH factor involved in neuronal specification and differentiation [Ito, H. et al., J Neurosci Res. 2003, 71, 648]. Altogether, these results provide compiling evidences indicating that compounds according to the invention promote NSCs differentiation, through the inhibition of Notch signaling.

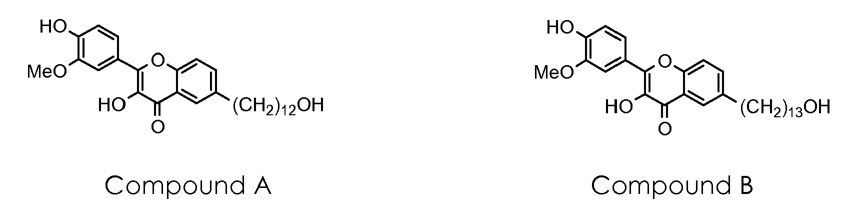

Fig. 2: Compound A and B induces NSCs differentiation into neurons. Real-time PCR gene expression analysis in neurospheres. RNA extraction was performed after 6 hours of incubation with compound A and B (1 µM) or with 0.1% DMSO vehicle as control. Expression of Mash1 transcripts in treated neurospheres are significantly increased with a decrease of Hes5 gene expression. β-actin was used as an internal control.

### Example 31: Differentiation of human neuroblastoma cell line SH-SY5Y into neurons

SH-SY5Y cells were cultured in 6 well plates at a density of 500000 cells per well and was maintained in DMEM with 10% FBS. After 24h, the cells were treated with test compounds (1 µM) or 0.1% DMSO vehicle as control. RNA extraction was performed after 24 hours of incubation and gene expression was analyzed by real-time PCR.

The specific primer pairs directed against human Hash1, Notch1 and beta-actin are from the online database qprimerdepot (http://primerdepot.nci.nih.gov/).

| **Gene (Accession No.)** | forward primer | reverse primer |
|---|---|---|
| **Beta-actin** *(NM*_*001101)* | 5'- GCACAGAGCCTCGCCTT- 3' | 5'- CCTTGCACATGCCGGAG-3' |
| **HASH1** *(NM*_*004316)* | 5'- GGAGCTTCTCGACTTCACC A-3' | 5'- CTAAAGATGCAGGTTGTGC G-3' |
| **Notch1** *(NM*_*017617)* | 5'- AGTGTGAAGCGGCCAATG -3' | 5'- ATAGTCTGCCACGCCTCTG- 3' |

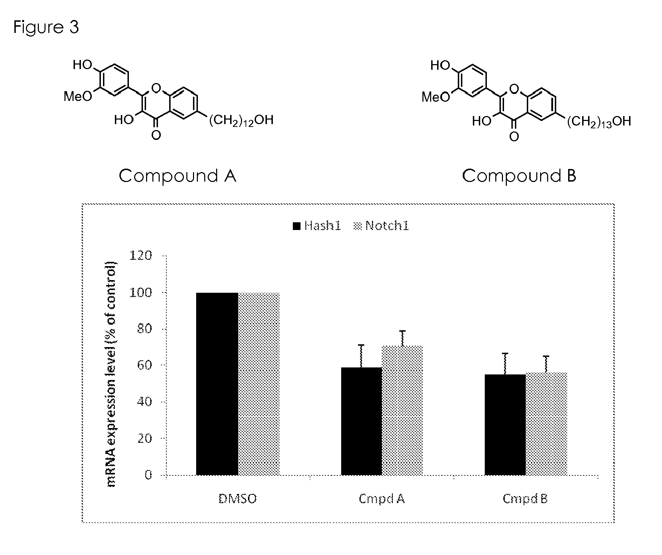

The human homolog of Mash1, Hash1, is expressed in neuroblastoma cell lines. However, Hash1 transcripts became downregulated in several cell lines during induced differentiation [Ichimiya, S. et al., Med Pediatr Oncol. 2001, 36,132]. Therefore, we tested whether compounds of general Formula (I) and (II) could induce the differentiation of SH-SY5Y neuroblastoma cell line by modulating the gene expression of Hash1 and Notch1. Fortunately, cultures treated with example compounds A and B at 1 µM (figure 3) proved to inhibit the expression of both Hash1 and Notch1 transcripts correlating with the differentiation of neuroblastoma cell lines into a neuronal phenotype.

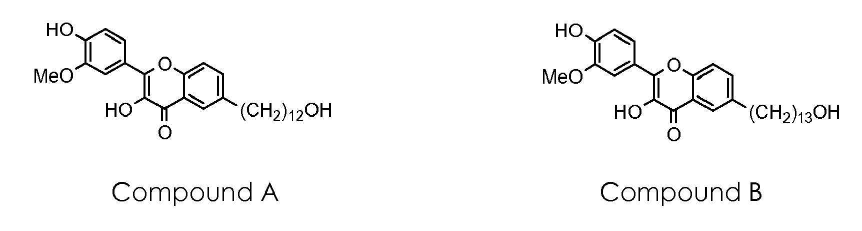

Fig. 3: Compound A and B induces neuroblastoma differentiation into a neuronal phenotype. Real-time PCR gene expression analysis in SH-SY5Y neuroblastoma cell line. RNA extraction was performed after 24 hours of incubation with compound A and B (1 µM) or with 0.1% DMSO vehicle as control. Expression of Hash1 and Notch1 transcripts in treated SH-SY5Y cell line are significantly decreased. β-actin was used as an internal control.

### References

Artavanis-Tsakonas, S., Rand, M.D., Lake, R.J., Notch signaling: cell fate control and signal integration in development. Science. 1999, 284(5415), 770-776.
Beher, D., Graham, S.L., Protease inhibitors as potential disease-modifying therapeutics for Alzheimer's disease. Expert Opin Investig Drugs. 2005, 14(11), 1385-1409.
Borg, J., Toazara, J., Hietter, H., Henry, M., Schmitt, G., Luu B., Neurotrophic effect of naturally occurring long-chain fatty alcohols on cultured CNS neurons. FEBS Lett. 1987, 213(2), 406-410.
Briend, E., Young, L.L., McKenzie, G.J., Tugal, T., Ragno, S., Champion, B.R., Modulation of the notch pathway for immunotherapy. Curr Opin Mol Ther. 2005, 7(1), 56-61.
Caldwell, M.A., He, X., Wilkie, N., Pollack, S., Marshall, G., Wafford, K.A., Svendsen, C.N., Growth factors regulate the survival and fate of cells derived from human neurospheres. Not Biotechnol. 2001, 19(5), 475-479.
Coowar, D., Bouissac, J., Hanbali, M., Paschaki, M., Mohier, E., Luu, B., Effects of indole fatty alcohols on the differentiation of neural stem cell derived neurospheres. J Med Chem. 2004, 47(25), 6270-6282.
Ekdahl, C.T., Claasen, J.H., Bonde, S., Kokaia, Z., Lindvall, O., Inflammation is detrimental for neurogenesis in adult brain. Proc Natl Acad Sci USA. 2003, 100(23), 13632-13637.
Ichimiya, S., Nimura, Y., Seki, N., Ozaki, T., Nagase, T., Nakagawara, A., Downregulation of hASH1 is associated with the retinoic acid-induced differentiation of human neuroblastoma cell lines. Med Pediatr Oncol. 2001, 36(1), 132-134.
Ito, H., Nakajima, A., Nomoto, H., Furukawa, S., Neurotrophins facilitate neuronal differentiation of cultured neural stem cells via induction of mRNA expression of basic helix-loop-helix transcription factors Mash1 and Math1. J Neurosci Res. 2003, 71 (5), 648-658.
John, G.R., Shankar, S.L., Shafit-Zagardo, B., Massimi, A., Lee, S.C., Raine, C.S., Brosnan, C.F., Multiple sclerosis: re-expression of a developmental pathway that restricts oligodendrocyte maturation. Not Med. 2002, 8(10), 1115-1121.
Klegeris, A., McGeer, E.G., McGeer, P.L., Therapeutic approaches to inflammation in neurodegenerative disease. Curr Opin Neurol. 2007, 20(3), 351-357.
Maher, P., Akaishi, T., Abe, K., Flavonoid fisetin promotes ERK-dependent long-term potentiation and enhances memory. Proc Natl Acad Sci USA. 2006, 103(44), 16568-16573.
Manthey, J.A., Grohmann, K., Guthrie, N., Biological properties of citrus flavonoids pertaining to cancer and inflammation. Curr Med Chem. 2001, 8(2), 135-153.
Martino, G., Pluchino, S., The therapeutic potential of neural stem cells. Nat Rev Neurosci. 2006, 7(5), 395-406.
Middleton, E. Jr, Kandaswami, C., Theoharides, T.C., The effects of plant flavonoids on mammalian cells: implications for inflammation, heart disease, and cancer. Pharmacol Rev. 2000, 52(4), 673-751.
Miele, L., Golde, T., Osborne, B., Notch signaling in cancer. Curr Mol Med. 2006, 6(8), 905-918.
Nagase, H., Yamakuni, T., Matsuzaki, K., Maruyama, Y., Kasahara, J., Hinohara, Y., Kondo, S., Mimaki, Y., Sashida, Y., Tank, A.W., Fukunaga, K., Ohizumi, Y., Mechanism of neurotrophic action of nobiletin in PC12D cells. Biochemistry. 2005, 44(42), 13683-13691.
Saxe, J.P., Wu, H., Kelly, T.K., Phelps, M.E., Sun, Y.E., Kornblum, H.I., Huang, J., A phenotypic small-molecule screen identifies an orphan ligand-receptor pair that regulates neural stem cell differentiation. Chem Biol. 2007, 14(9), 1019-1030.
Warashina, M., Min, K.H., Kuwabara, T., Huynh, A., Gage, F.H., Schultz, P.G., Ding, S., A synthetic small molecule that induces neuronal differentiation of adult hippocampal neural progenitor cells. Angew Chem Int Ed Engl. 2006, 45(4), 591-593.
Zhao, C., Deng, W., Gage, F.H., Mechanisms and functional implications of adult neurogenesis. Cell. 2008, 132(4), 645-660.

## Claims

1. A compound of the general formula (I) or (II) 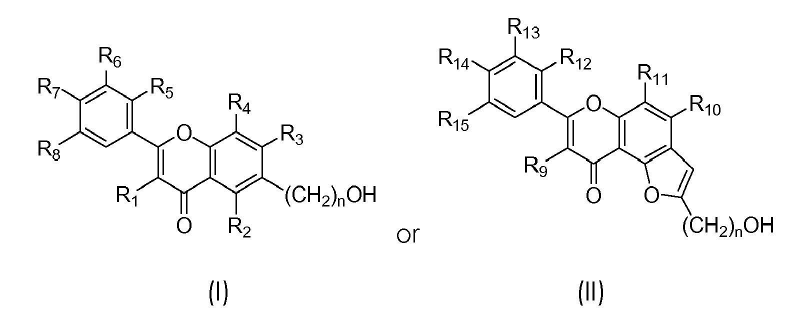 or a pharmaceutically acceptable salt thereof, wherein each of R₁, R₂, R₃, R₄, R₅, R₆, R₇, R₈, R₉, R₁₀, R₁₁, R₁₂, R₁₃, R₁₄ and R₁₅ independently represents a hydrogen atom, a hydroxyl group, a (C₁-C₆) alkyl group, a (C₂-C₆) alkenyl group, a (C₂-C₆) alkynyl group, a (C₁-C₆) alkoxy group, a hydroxy(C₁-C₆) alkyl group, a (C₁-C₆) alkoxy(C₁-C₆) alkyl group, a (C₁-C₆) alkanoyloxy group, a (C₁-C₆) alkanoyl group, a carboxyl group, a (C₁-C₆) alkoxycarbonyl group, a carbamoyl group, a (C₁-C₆) alkylaminocarbonyl group, a di(C₁-C₆) alkylaminocarbonyl group, a halogen atom, a halo(C₁-C₆) alkyl group, a halo(C₁-C₆) alkoxy group, a nitrile group, an amino group, a (C₁-C₆) alkylamino group, a di(C₁-C₆) alkylamino group or a (C₁-C₆) alkanoylamino group, or two adjacent groups represent a methylenedioxy group, and n is an integer in the range of from 8 to 25.

2. A compound as claimed in claim 1, in which n in formula (I) is an integer in the range of from 10 to 20 and n in formula (II) is an integer in the range of from 8 to 18.

3. A compound as claimed in claim 1, in which n is 9, 10, 11, 12, 13 or 14 or 15.

4. A compound as claimed in any one of claims 1 to 3, in which each of R₁, R₂, R₃, R₄, R₅, R₆, R₇, R₈, R₉, R₁₀, R₁₁, R₁₂, R₁₃, R₁₄ and R₁₅ independently represents a hydrogen atom, a hydroxyl group or a (C₁-C₆) alkoxy group.

5. A compound as claimed in any one of claims 1 to 4, wherein each of R₄ and R₁₁ represents a hydrogen atom.

6. A compound as claimed in any one of claims 1 to 5, in which each of R₇ and R₁₄ represents a hydroxy group or a (C₁-C₆) alkoxy group.

7. A compound as claimed in any one of claims 1 to 3, 5 and 6, wherein each of each of R₅ and R₁₂ represents a hydrogen atom, a hydroxyl group or a (C₁-C₆) alkoxy group.

8. A compound as claimed in any one of claims 1 to 3, and 5 to 7, wherein each of R₁ and R₉ represents a hydrogen atom, a hydroxy group or a (C₁-C₆) alkoxy group.

9. A compound as claimed in claim 8, wherein each of R₁ and R₉ represents a hydrogen atom or a hydroxy group.

10. A compound as claimed in any one of claims 1 to 3 and 5 to 9, wherein each of R₂, R₃ and R₁₀ represents a hydrogen atom, a hydroxy group or a (C₁-C₆) alkoxy group.

11. A compound as claimed in any one of claims 1 to 3 and 5 to 10, wherein each of R₈ and R₁₅ represents a hydrogen atom, a hydroxy group or a (C₁-C₆) alkoxy group.

12. A process for the preparation of a compound as claimed in any one of claims 1 to 11, which comprises
a) for a compound of formula (II), reacting a compound of the general formula (III) 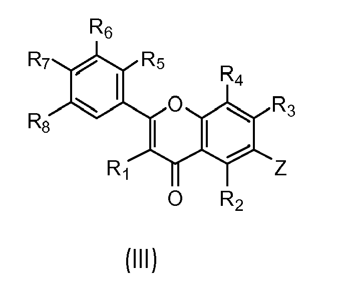 or a protected derivative thereof, in which Z represents a leaving atom or group with a compound of formula 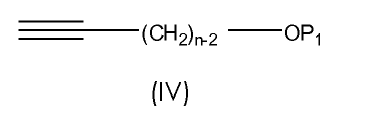 in which P₁ represents a hydrogen atom or a hydroxyl protecting group in the presence of a Group VIII metal catalyst under cyclisation conditions;
b) for a compound of formula (I), reducing a compound of the general formula (V) 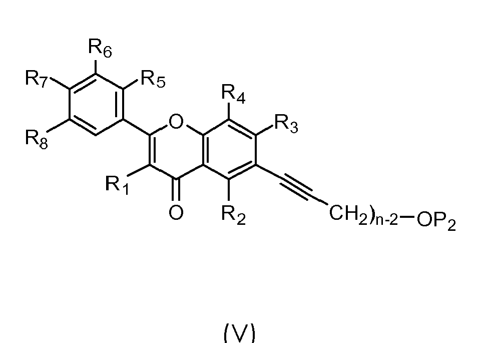 or a protected derivative thereof, in which P₂ represents a hydrogen atom or a hydroxyl protecting group;
followed by removing any protecting groups and if desired, forming a pharmaceutically acceptable salt.

13. A pharmaceutical composition, which comprises a compound as claimed in any one of claims 1 to 11 and a pharmaceutically acceptable carrier.

14. A compound as claimed in any one of claims 1 to 11, for use in therapy.

15. A compound as claimed in any one of claims 1 to 11, for use in the treatment of a neurodegenerative disease.

16. A method of treating a neurodegenerative disease in a patient requiring treatment, which comprises administering an effective amount of a compound as claimed in claim 1.

17. A compound of general formula (III) 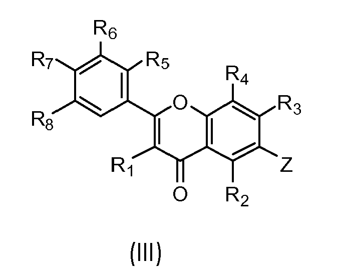 in which Z represents a leaving atom or group and each of R₁, R₂, R₃, R₄, R₅, R₆, R₇ and R₈ is as defined in claim 1, or a salt or protected derivative thereof,.

18. A compound of general formula (V) 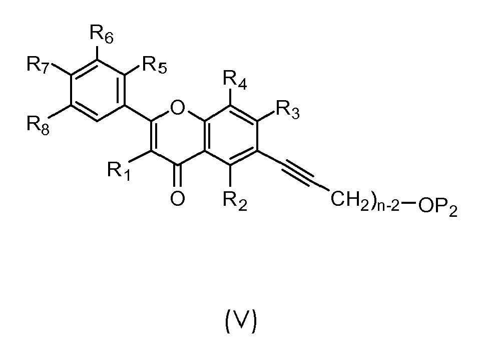 in which P₂ represents a hydrogen atom or a hydroxyl protecting group and each of R₉, R₁₀, R₁₁, R₁₂, R₁₃, R₁₄ and R₁₅ or a salt or protected derivative thereof.
